# EUROPEAN PATENT APPLICATION

(11) **EP 4 610 258 A1**
(43) Date of publication of application: **03.09.2025**
(21) Application number: 23882674.7
(22) Date of filing: 25.10.2023
(51) Int. Cl.: C07D 471/04, C07D 487/04, A61P 25/28, A61P 29/00, C07D 498/04, C07D 513/04, A61K 31/5025, A61K 31/519, A61K 31/551, A61K 31/553, A61K 31/554

(54) **FUSED PYRIDAZINE DERIVATIVE**

(30) Priority: 26.10.2022 JP 2022171096
(71) Applicant: Tenvie Therapeutics, Inc., Chicago, Illinois 60631 (US)
(72) Inventor: INAGAKI, Yusuke, Tokyo 103-8411 (JP); KOIZUMI, Yuka, Tokyo 103-8411 (JP); WASHIO, Takuya, Tokyo 103-8411 (JP); YAMASHITA, Yumi, Tokyo 103-8411 (JP); TOYA, Hiroki, Tokyo 103-8411 (JP); IIKUBO, Kazuhiko, Tokyo 103-8411 (JP); TOMIYAMA, Hiroshi, Hanishina-gun, Nagano 389-0606 (JP); IWAI, Yoshinori, Hanishina-gun, Nagano 389-0606 (JP); NAKAMURA, Akihiko, Hanishina-gun, Nagano 389-0606 (JP)
(74) Representative: Gille Hrabal Partnerschaftsgesellschaft mbB Patentanwälte
(86) International application number: PCT/JP2023/038490
(87) International publication number: WO 2024/090469

(57) **Abstract**

An object of the present invention is to provide a pharmaceutical composition, in particular, a compound suitable for preventing and/or treating inflammatory diseases and/or neurodegenerative diseases. The present inventors have conducted intensive studies to find a compound having an inhibitory effect on NLRP3 inflammasome activation and found that an annulated pyridazine derivative has an inhibitory effect on NLRP3 inflammasome activation, thus completing the present invention. The annulated pyridazine derivative of the present invention is expected to serve as a prophylactic and/or therapeutic agent for inflammatory diseases and/or neurodegenerative diseases.

## Description

### TECHNICAL FIELD

The present invention relates to an annulated pyridazine derivative having an inhibitory effect on NLRP3 inflammasome activation, which derivative is expected to be useful as an active ingredient of a pharmaceutical composition, for example, a pharmaceutical composition for preventing and/or treating inflammatory diseases and/or neurodegenerative diseases, or a salt thereof.

### BACKGROUND ART

An inflammasome is an assembly of intracellular proteins induced by endogenous and exogenous alarm molecules and a mechanism responsible for amplification of inflammatory responses through activation caused by cleavage of inflammatory cytokines IL-1β and IL-18 via activation of caspase-1, as well as induction of cell death. A plurality of types of molecules recognizing alarm molecules are known, which include NLRP1, NLRP3, NLRC4, and AIM2, and NLRP3 is activated by recognizing cellular stress caused by, for example, an extracellular ATP molecule, a toxin of a pathogen, a crystal of uric acid or cholesterol, and an abnormal aggregate of proteins.

As a disease cause by NLRP3 gain-of-function mutations, cryopyrin-associated periodic syndrome (CAPS) is known (Nature Genetics, Vol. 29, No. 3, pp. 301-305, 2001). Moreover, it is reported that NLRP3 inflammasomes are activated or highly expressed in a wide variety of diseases such as gout (Arthritis Research and Therapy, Vol. 12, No. 2, Article No. 206, 2010), nonalcoholic steatohepatitis (Journal of Molecular Medicine, Vol. 92, No. 10, pp. 1069-1082, 2014), inflammatory bowel disease (Gut, Vol. 59, No. 9, pp. 1192-1100, 2010), Alzheimer's disease (Nature, Vol. 493, No. 7434, pp. 674-678, 2013), Parkinson's disease (PLoS ONE, Vol. 8, No. 1, Article No. e55375, 2013), amyotrophic lateral sclerosis (Inflammation, Vol. 41, No. 1, pp. 93-103, 2018), and multiple system atrophy (Journal of Neuropathology and Exprimental Neurology, Vol. 77, No. 11, pp. 1055-1065, 2018).

It is also known that α-synuclein fibrils activate NLRP3 to promote IL-1β production from microglia; and that administration of an NLRP3 inhibitor improves functions in a mouse model with α-synucleinopathy induced by α-synuclein fibrils (Science Translational Medicine, Vol. 10, Article No. eaah4066, 2018).

PTL 1 describes that a compound represented by the following formula has an inhibitory effect on the NLRP3 inflammasome pathway (see the publication for symbols in the formula).

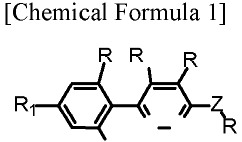

PTL 2 describes that a compound represented by the following formula has an inhibitory effect on NLRP3 inflammasome activation (see the publication for symbols in the formula).

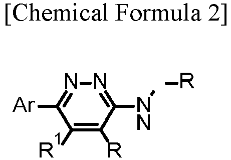

PTL 3 describes that a compound represented by the following formula has an NLRP3 inhibitory effect (see the publication for symbols in the formula).

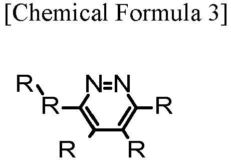

PTL 4 describes that a compound represented by the following formula has an inhibitory effect on the NLRP3 inflammasome (see the publication for symbols in the formula).

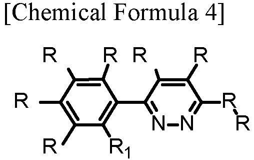

PTL 5 describes that a compound represented by the following formula has an NLRP3 inhibitory effect (see the publication for symbols in the formula).

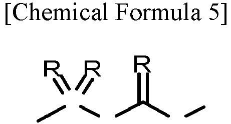

PTL 6 describes that a compound represented by the following formula has an NLRP1/3 modulating effect (see the publication for symbols in the formula).

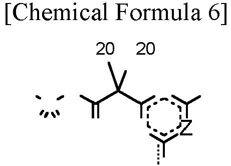

PTL 7 describes that a compound represented by the following formula has an inhibitory effect on the NLRP3 inflammasome (see the publication for symbols in the formula).

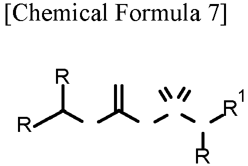

PTL 8 describes that a compound represented by the following formula has a splicing regulatory effect (see the publication for symbols in the formula).

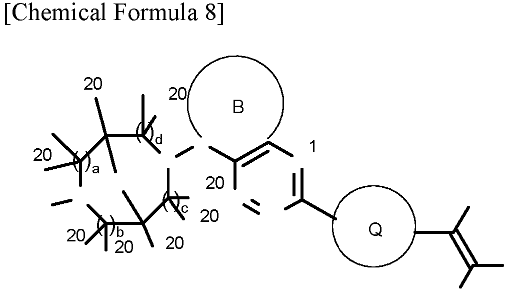

PTL 9 describes that a compound represented by the following formula has a splicing regulatory effect (see the publication for symbols in the formula).

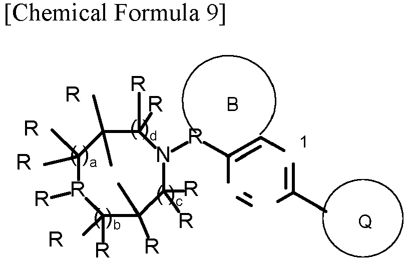

PTL 10 describes that a compound represented by the following formula has a splicing regulatory effect (see the publication for symbols in the formula).

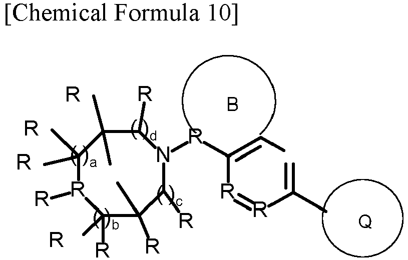

PTL 11 describes that a compound represented by the following formula has an effect of treating Huntington's disease (see the publication for symbols in the formula).

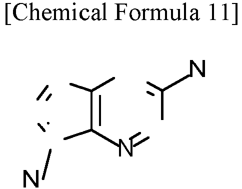

### CITATION LIST

### PATENT LITERATURE

PTL 1: International Publication No. WO 2020/234715
PTL 2: International Publication No. WO 2021/193897
PTL 3: U.S. Patent No. 11319319
PTL 4: International Publication No. WO 2022/135567
PTL 5: International Publication No. WO 2019/008025
PTL 6: International Publication No. WO 2017/184604
PTL 7: International Publication No. WO 2018/015445
PTL 8: International Publication No. WO 2022/060951
PTL 9: International Publication No. WO 2022/060943
PTL 10: International Publication No. WO 2020/190793
PTL 11: International Publication No. WO 2020/005873

### SUMMARY OF INVENTION

### TECHNICAL PROBLEM

Provided is a compound having an inhibitory effect on NLRP3 inflammasome activation, which compound is expected to be useful as an active ingredient of a pharmaceutical composition, in particular, a pharmaceutical composition for preventing and/or treating, for example, inflammatory diseases and/or neurodegenerative diseases.

### SOLUTION TO PROBLEM

As a result of intensive studies on a compound having an inhibitory effect on NLRP3 inflammasome activation, the present inventors have found that an annulated pyridazine derivative has an inhibitory effect on NLRP3 inflammasome activation, which derivative is expected to be useful as an active ingredient of a pharmaceutical composition for preventing and/or treating, for example, inflammatory diseases and/or neurodegenerative diseases, thus completing the present invention.

In other words, the present invention relates to a compound of formula (I) or a salt thereof and a pharmaceutical composition containing the compound of formula (I) or a salt thereof and one or more excipients. 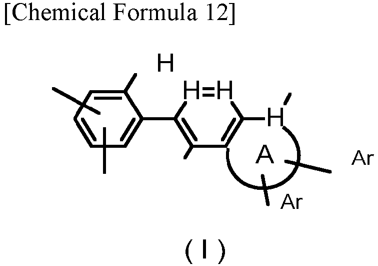 wherein
ring A is 5- to 7-membered nitrogen-containing partially unsaturated heterocycle,
R^{1a} and R^{1b} are each independently H, C₁₋₆ alkyl, -O-C₁₋₆ alkyl, halogeno C₁₋₆ alkyl, -O-halogeno C₁₋₆ alkyl, C₃₋₆ cycloalkyl, -O-C₃₋₆ cycloalkyl, or halogen,
R² is H or C₁₋₆ alkyl,
R^{3a} and R^{3b} are each independently H, C₁₋₆ alkyl, -O-C₁₋₆ alkyl, -C₁₋₆ alkylene-optionally substituted aryl, oxo, -C(=O)-C₁₋₆ alkyl, or -S(=O)₂-C₁₋₆ alkyl,
R⁴ is C₁₋₆ alkyl substituted with one to three R⁵, C₃₋₈ cycloalkyl optionally substituted with one to four R⁶, 4- to 7-membered saturated heterocyclyl optionally substituted with one to four R⁷, or heteroaryl optionally substituted with one to four R⁸,
R⁵, R⁶, R⁷, and R⁸ are each independently C₁₋₆ alkyl, C₃₋₈ cycloalkyl, halogeno C₁₋₆ alkyl, 4- to 7-membered saturated heterocyclyl, oxo, cyano, -C(=O)-C₁₋₆ alkyl, -S(=O)₂-C₁₋₆ alkyl, - C(=O)-O-C₁₋₆ alkyl, -C(=O)-NR⁹R¹⁰, -NR⁹R¹⁰, -C₁₋₆ alkylene-OR¹¹, or -OR¹¹,
R⁹ and R¹⁰ are each independently H, C₁₋₆ alkyl, or -C(=O)-C₁₋₆ alkyl, and
R¹¹ is H, C₁₋₆ alkyl, or -C(=O)-C₁₋₆ alkyl.

Note that the substituents R^{3a} and R^{3b} in the formula are each bonded to an atom, namely an atom constituting ring A, excluding the two carbon atoms shared with the pyridazine ring and the nitrogen atom attached to R⁴. In addition, unless otherwise noted, when a symbol in one chemical formula herein is used in another chemical formula, the same symbol has the same meaning.

The present invention also relates to a pharmaceutical composition containing the compound of formula (I) or a salt thereof and one or more pharmaceutically acceptable excipients, in particular, a pharmaceutical composition for preventing and/or treating inflammatory diseases and/or neurodegenerative diseases. Note that the pharmaceutical composition includes a prophylactic and/or therapeutic agent for inflammatory diseases and/or neurodegenerative diseases, the agent containing the compound of formula (I) or a salt thereof.

In addition, the present invention relates to a pharmaceutical composition containing the compound of formula (I) or a salt thereof, which is an inhibitor of NLRP3 inflammasome activation; the compound of formula (I) or a salt thereof for use as an inhibitor of NLRP3 inflammasome activation; an inhibitor of NLRP3 inflammasome activation, the inhibitor containing the compound of formula (I) or a salt thereof; a pharmaceutical composition containing the compound of formula (I) or a salt thereof, which is an inhibitor of NLRP3 inflammasome activation, and one or more pharmaceutically acceptable excipients; use of the compound of formula (I) or a salt thereof for the manufacture of a medicament or a pharmaceutical composition for preventing and/or treating an inflammatory disease and/or neurodegenerative disease; use of the compound of formula (I) or a salt thereof for prevention and/or treatment of an inflammatory disease and/or neurodegenerative disease; the compound of formula (I) or a salt thereof for use in prevention and/or treatment of an inflammatory disease and/or neurodegenerative disease; and a method for preventing and/or treating an inflammatory disease and/or neurodegenerative disease, including administering an effective amount of the compound of formula (I) or a salt thereof to a subject. Note that the "subject" is a human or a non-human animal in need of the prevention and/or treatment, and in an aspect, is a human in need of the prevention and/or treatment.

### ADVANTAGEOUS EFFECTS OF INVENTION

The compound of formula (I) or a salt thereof has an inhibitory effect on NLRP3 inflammasome activation, and can be used as a prophylactic and/or therapeutic agent, for example, for inflammatory diseases and/or neurodegenerative diseases.

### DESCRIPTION OF EMBODIMENTS

Hereinafter, the invention will be described in detail.

In the present specification, the following terms, unless otherwise specified, have the following meanings. The following definitions are intended to clarify, but not limit, the terms defined. If a term used herein is not specifically defined, such a term is used in a sense generally accepted by those skilled in the art.

As used herein, the term "C₁₋₆ alkyl" refers to a linear or branched saturated hydrocarbon group having 1 to 6 carbon atoms (hereinafter abbreviated as C₁₋₆ as the carbon number expression), such as methyl, ethyl, n-propyl, isopropyl, n-butyl, isobutyl, sec-butyl, tert-butyl, n-pentyl, 1-methylbutyl, 2-methylbutyl, 3-methylbutyl, 1,1-dimethylpropyl, 2,2-dimethylpropyl, 1,2-dimethylpropyl, 1-ethylpropyl, n-hexyl, 1,3-dimethylbutyl, and 1-ethyl-2-methylpropyl. The C₁₋₆ alkyl is linear or branched C₁₋₄ alkyl in an aspect; methyl, ethyl, n-propyl, isopropyl, n-butyl, isobutyl, or tert-butyl in an aspect; methyl, ethyl, n-propyl, or n-butyl in an aspect; n-propyl or n-butyl in an aspect; n-propyl in an aspect; n-butyl in an aspect; methyl or ethyl in an aspect; ethyl in an aspect; or methyl in an aspect.

The term "C₁₋₆ alkylene" refers to a linear or branched C₁₋₆ divalent saturated hydrocarbon group, such as methylene, ethylene, trimethylene, tetramethylene, pentamethylene, hexamethylene, methylmethylene, 1-methylethylene, 2-methylethylene, 1,1-dimethylethylene, 2,2-dimethylethylene, 2-methyltrimethylene, ethylethylene, 1,2-dimethylethylene, and 1,1,2,2-tetramethylethylene. The C₁₋₆ alkylene is C₁₋₄ alkylene in an aspect; methylene, ethylene, trimethylene, 1,1-dimethylethylene, or 2,2-dimethylethylene in an aspect; ethylene, trimethylene, 1,1-dimethylethylene, or 2,2-dimethylethylene in an aspect; methylene or ethylene in an aspect; ethylene in an aspect; trimethylene in an aspect; 1,1-dimethylethylene in an aspect; 2,2-dimethylethylene in an aspect; or methylene in an aspect.

The term "C₃₋₈ cycloalkyl" refers to a C₃₋₈ saturated hydrocarbon ring group, which may have a cross-link or form a spiro ring, such as cyclopropyl, cyclobutyl, cyclopentyl, cyclohexyl, cycloheptyl, cyclooctyl, bicyclo[1.1.1]pentyl, bicyclo[3.1.0]hexyl, bicyclo[2.2.1]heptyl, bicyclo[3.1.1]heptyl, bicyclo[3.3.0]octyl, bicyclo[2.2.2]octyl, spiro[2.2]pentyl, spiro[3.3]heptyl, and spiro[2.5]octyl. The C₃₋₈ cycloalkyl is a C₃₋₆ saturated hydrocarbon ring group in an aspect; cyclopropyl, cyclobutyl, cyclopentyl, or cyclohexyl in an aspect; cyclopropyl in an aspect; cyclobutyl, cyclopentyl, or cyclohexyl in an aspect; cyclobutyl in an aspect; cyclopentyl in an aspect; or cyclohexyl in an aspect.

A "5- to 7-membered nitrogen-containing partially unsaturated heterocycle" refers to a 5- to 7-membered saturated hydrocarbon ring in which the ring has one carbon-carbon double bond, contains one nitrogen atom as a ring constituent atom, and may also contain one or more heteroatoms, in particular oxygen, nitrogen, or sulfur atoms as ring constituent atoms. The 5- to 7-membered nitrogen-containing partially unsaturated heterocycle is, for example, dihydropyrrole, tetrahydropyridine, tetrahydroazepine, dihydrooxazole, dihydroimidazole, dihydrothiazole, dihydrooxazine, tetrahydropyrazine, dihydrothiazine, tetrahydropyrimidine, tetrahydrooxaazepine, tetrahydrodiazepine, or tetrahydrothiazepine. The 5- to 7-membered nitrogen-containing partially unsaturated heterocycle is dihydropyrrole, tetrahydropyridine, tetrahydroazepine, tetrahydropyrimidine, tetrahydrooxaazepine, tetrahydrodiazepine or tetrahydrothiazepine in an aspect; or dihydropyrrole, tetrahydropyridine, tetrahydrooxaazepine, or tetrahydrodiazepine in an aspect.

In the following substructure of formula (I): 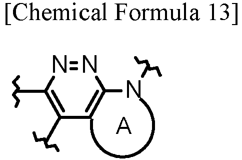 wherein wavy lines each indicate a site of bonding to the phenyl group, R², or R⁴ in formula (I), respectively; and the same applies to the following,
ring A, a 5- to 7-membered nitrogen-containing partially unsaturated heterocycle, shares its double bond moiety and condenses with the pyridazine ring to form, for example, the following substructures without limitation.

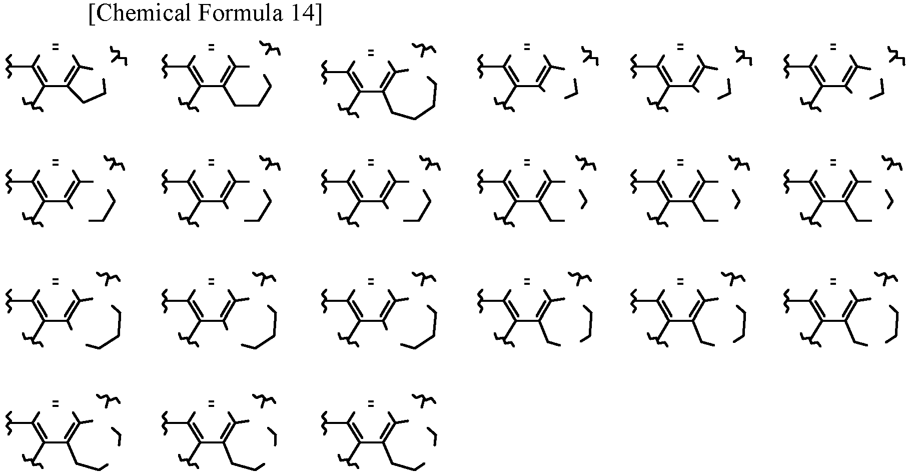

The term "4- to 7-membered saturated heterocyclyl" is a 4- to 7-membered saturated hydrocarbon ring group containing one or more hetero atoms, in particular, oxygen atoms, nitrogen atoms, or sulfur atoms as ring-constituting atoms, and examples thereof include, but are not limited to, oxetanyl, azetidinyl, thietanyl, tetrahydrofuranyl, pyrrolidinyl, tetrahydrothiophenyl, tetrahydropyranyl, piperidinyl, tetrahydrothiopyranyl, oxepanyl, azepanyl, thiepanyl, dioxolanyl, imidazolidinyl, pyrazolidinyl, dithiolanyl, oxazolidinyl, isoxazolidinyl, thiazolidinyl, isothiazolidinyl, dioxanyl, piperazinyl, dithianyl, morpholinyl, thiomorpholinyl, oxathialanyl, dioxepanyl, diazepanyl, dithiepanyl, oxazepanyl, thieazepanyl, and oxathiepanyl. The 4- to 7-membered saturated heterocyclyl is oxetanyl, azetidinyl, tetrahydrofuranyl, pyrrolidinyl, tetrahydropyranyl, piperidinyl, oxetanyl, or azepanyl in an aspect; tetrahydrofuranyl, pyrrolidinyl, tetrahydropyranyl, or piperidinyl in another aspect; oxetanyl, pyrrolidinyl, tetrahydropyranyl, or piperidinyl in still another aspect; pyrrolidinyl, tetrahydropyranyl, or piperidinyl in still another aspect; tetrahydropyranyl in still another aspect; piperidinyl in still another aspect; oxetanyl or tetrahydropyranyl in another aspect; or oxetanyl in still another aspect.

The term "aryl" refers to a C₆₋₁₄ mono- to tricyclic aromatic hydrocarbon group, which includes a bi- to tricyclic aromatic hydrocarbon group fused with C₅₋₈ cycloalkene at a double-bond site thereof, such as, phenyl, naphthyl, tetrahydronaphthyl, indenyl, and fluorenyl. In an aspect, the aryl is phenyl.

The term "heteroaryl" refers to a 5 or 6-membered aromatic hydrocarbon ring group containing 1 to 4 hetero atoms selected from oxygen, sulfur, and nitrogen, such as pyrrolyl, furyl, thienyl, pyrazolyl, imidazolyl, isoxazolyl, oxazolyl, isothiazolyl, thiazolyl, triazolyl, oxadiazolyl, thiadiazolyl, pyridyl, pyrazinyl, pyrimidinyl, pyridazinyl, triazinyl, and tetrazinyl. The heteroaryl is pyrazolyl in an aspect.

The term "halogen" means F, Cl, Br, or I. The halogen is F or Cl in an aspect; F in another aspect; or Cl in still another aspect.

The term "halogeno C₁₋₆ alkyl" refers to a linear or branched C₁₋₆ alkyl group substituted with one or more halogen atoms. Examples thereof include trifluoromethyl, trifluoroethyl, trifluoropropyl, 2-fluoro-2-methylpropyl, difluoromethyl, difluoroethyl, fluoromethyl, and chloromethyl. The halogeno C₁₋₆ alkyl is difluoromethyl, difluoroethyl, trifluoromethyl, or trifluoroethyl in an aspect; difluoromethyl, difluoroethyl, or trifluoromethyl in another aspect; trifluoromethyl or difluoromethyl in still another aspect; trifluoromethyl in an aspect; difluoromethyl in another aspect; difluoroethyl or trifluoroethyl in another aspect; difluoroethyl in an aspect; or trifluoroethyl in a further aspect.

As used herein, "optionally substituted" means either unsubstituted or "substituted with one or more substituents (e.g., substituents defined below)". The substitution may occur at any position where hydrogen is normally present in the group of interest. In an aspect, the wording "optionally substituted" refers to "optionally substituted with 1 to 4 substituents"; in another aspect, "optionally substituted with 1 to 3 substituents". Note that in the case of multiple substitutions, the substituents may be the same or different. Examples of the specific substituent include C₁₋₆ alkyl, -O-C₁₋₆ alkyl, halogeno C₁₋₆ alkyl, -O-halogeno C₁₋₆ alkyl, C₃₋₈ cycloalkyl, -O-C₃₋₈ cycloalkyl, cyano, oxo, and halogen. In an aspect, the substituent is -O-C₁₋₆ alkyl.

The wording "optionally substituted aryl" means aryl unsubstituted or substituted with one or more substituents, in an aspect, phenyl unsubstituted or substituted with one or more substituents, and in another aspect, phenyl unsubstituted or substituted with -O-C₁₋₆ alkyl.

Even if a combination is not specifically described, one or more aspects may be combined with another aspect.

As used herein, the term "inflammatory disease and/or neurodegenerative disease" refers to an inflammatory disease and a neurodegenerative disease in an aspect; an inflammatory disease in a further aspect; and a neurodegenerative disease in an aspect.

As used herein, the term "inflammatory disease" refers to, but is not limited to, an autoinflammatory disease including cryopyrin-associated periodic syndrome (CAPS), gout, and pseudogout; and a disease including nonalcoholic steatohepatitis (NASH). The inflammatory disease is an autoinflammatory disease in an aspect; CAPS in another aspect; and gout in yet another aspect. Note that the term "cryopyrin-associated periodic syndrome (CAPS)" refers to a disease selected from the group consisting of the familial cold urticaria (FCAS), Muckle-Wells syndrome (MWS), and neonatal onset multisystem inflammatory diseases/chronic infantile neurologic cutaneous and articular syndrome (NOMID/CINCA syndrome).

As used herein, the term "neurodegenerative disease" refers to a group of diseases including, but not limited to, α-synucleinopathies including Parkinson's disease, multiple system atrophy, and Lewy body dementia; Alzheimer's disease; amyotrophic lateral sclerosis; and multiple sclerosis. The neurodegenerative disease is Alzheimer's disease, multiple sclerosis, and amyotrophic lateral sclerosis in an aspect; or multiple sclerosis in a further aspect. The neurodegenerative disease is α-synucleinopathy in another aspect; Parkinson's disease in yet another aspect; multiple system atrophy in still another aspect; and Lewy body dementia in still another aspect.

As used herein, the term "treatment" includes both "treatment for therapeutic purposes" and "treatment for prophylactic purposes". The term "treatment for therapeutic purposes" means, for example, alleviating symptoms, altering the course of a disease, and prolonging life, and the term "treatment for prophylactic purposes" means reducing the likelihood of developing a disease in a subject at risk of developing the disease. The "subject at risk of developing the disease" means an individual with known risk factors who is more likely to develop the disease than the general population.

Embodiments of the compound of formula (I) or a salt thereof in the present invention are shown below.
(1-1) A compound of formula (I) or a salt thereof (ring A is 5- to 7-membered nitrogen-containing partially unsaturated heterocycle) 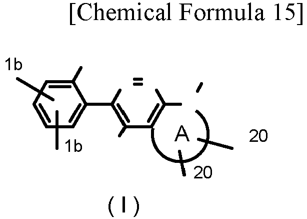
(1-2) A compound of formula (Ia), (Ib), (Ic), (Id), (Ie), (If), or (Ig), or a salt thereof. 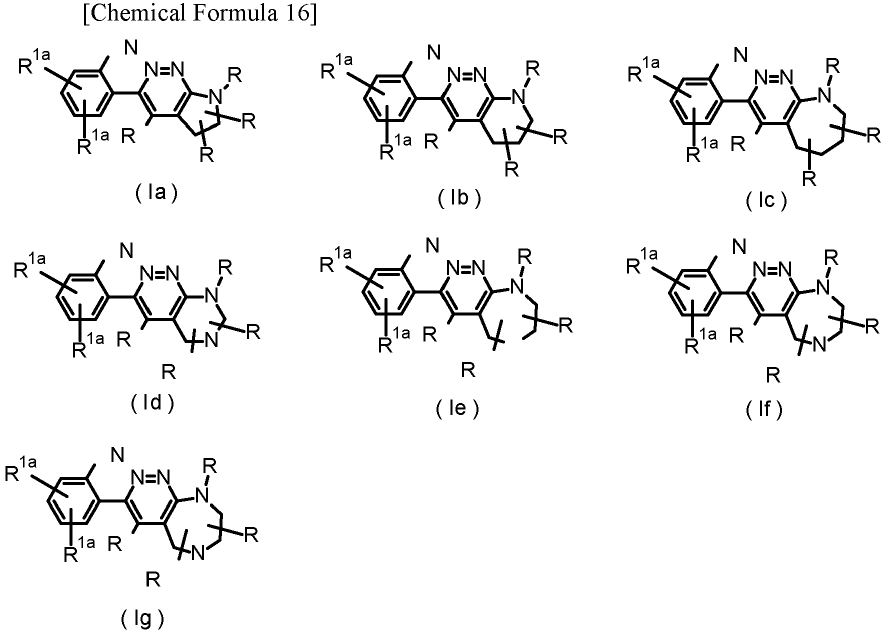
(1-3) A compound of formula (Ia), (Ib), (Ic), (Id), (Ie), or (If), or a salt thereof.
(1-4) A compound of formula (Ia), (Ib), (Ie), or (If), or a salt thereof.
(1-5) A compound of formula (Ia) or a salt thereof.
(1-6) A compound of formula (Ib) or a salt thereof.
(1-7) A compound of formula (Ic) or a salt thereof.
(1-8) A compound of formula (Id) or a salt thereof.
(1-9) A compound of formula (Ie) or a salt thereof.
(1-10) A compound of formula (If) or a salt thereof.
(1-11) A compound of formula (Ig) or a salt thereof.
(1-12) A compound of formula (Ia'), (Ib'), (Ic'), (Id'), (Ie'), (If'), or (Ig'), or a salt thereof. 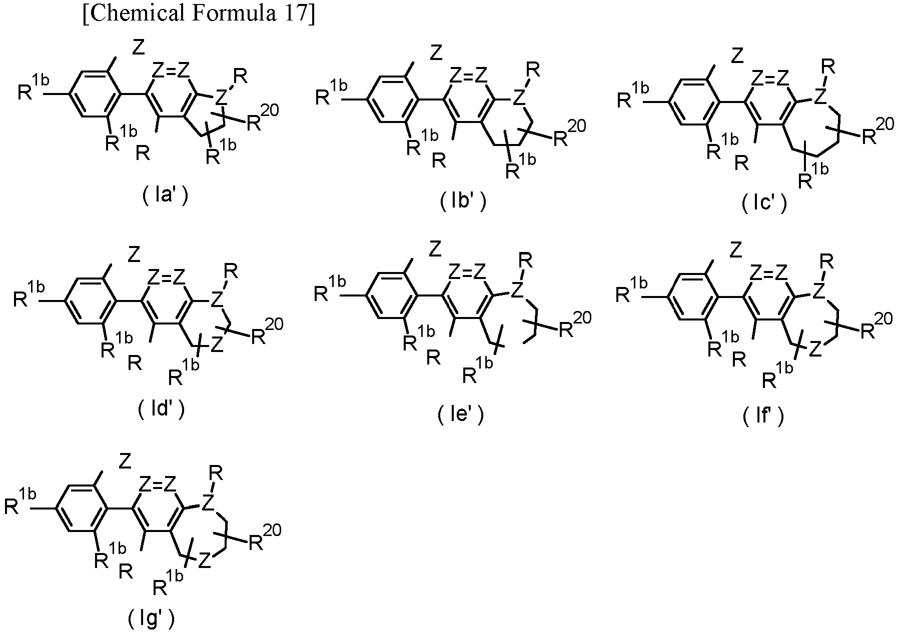
(1-13) A compound of formula (Ia'), (Ib'), (Ic'), (Id'), (Ie'), or (If), or a salt thereof.
(1-14) A compounds of formula (Ia'), (Ib'), (Ie'), or (If'), or a salt thereof.
(1-15) A compound of formula (Ia') or a salt thereof.
(1-16) A compound of formula (Ib') or a salt thereof.
(1-17) A compound of formula (Ic') or a salt thereof.
(1-18) A compound of formula (Id') or a salt thereof.
(1-19) A compound of formula (Ie') or a salt thereof.
(1-20) A compound of formula (If) or a salt thereof.
(1-21) A compound of formula (Ig') or a salt thereof.
(2-1) The compound or a salt thereof, wherein R^{1a} and R^{1b} are each independently H, C₁₋₆ alkyl, -O-C₁₋₆ alkyl, halogeno C₁₋₆ alkyl, -O-halogeno C₁₋₆ alkyl, C₃₋₆ cycloalkyl, -O-C₃₋₆ cycloalkyl, or halogen.
(2-2) The compound or a salt thereof, wherein R^{1a} and R^{1b} are each independently H, C₁₋₆ alkyl, -O-C₁₋₆ alkyl, halogeno C₁₋₆ alkyl, -O-halogeno C₁₋₆ alkyl, or halogen.
(2-3) The compound or a salt thereof, wherein R^{1a} is C₁₋₆ alkyl, -O-C₁₋₆ alkyl, halogeno C₁₋₆ alkyl, -O-halogeno C₁₋₆ alkyl, or halogen and R^{1b} is H or C₁₋₆ alkyl.
(2-4) The compound or a salt thereof, wherein R^{1a} is C₁₋₆ alkyl, -O-C₁₋₆ alkyl, halogeno C₁₋₆ alkyl, -O-halogeno C₁₋₆ alkyl, or halogen and R^{1b} is H.
(2-5) The compound or a salt thereof, wherein R^{1a} is -O-C₁₋₆ alkyl, halogeno C₁₋₆ alkyl, or -O-halogeno C₁₋₆ alkyl and R^{1b} is H.
(2-6) The compound or a salt thereof, wherein R^{1a} is -O-C₁₋₆ alkyl and R^{1b} is H.
(2-7) The compound or a salt thereof, wherein R^{1a} is halogeno C₁₋₆ alkyl and R^{1b} is H.
(2-8) The compound or a salt thereof, wherein R^{1a} is -O-halogeno C₁₋₆ alkyl and R^{1b} is H.
(2-9) The compound or a salt thereof, wherein R^{1a} is halogeno C₁₋₆ alkyl or -O-halogeno C₁₋₆ alkyl and R^{1b} is H.
(3-1) The compound or a salt thereof, wherein R² is H or C₁₋₆ alkyl.
(3-2) The compound or a salt thereof, wherein R² is H.
(3-3) The compound or a salt thereof, wherein R² is C₁₋₆ alkyl.
(4-1) The compound or a salt thereof, wherein R^{3a} and R^{3b} are each independently H, C₁₋₆ alkyl, -O-C₁₋₆ alkyl, -C₁₋₆ alkylene-optionally substituted aryl, oxo, -C(=O)-C₁₋₆ alkyl, or - S(=O)₂-C₁₋₆ alkyl.
(4-2) The compound or a salt thereof, wherein R^{3a} and R^{3b} are each independently H, C₁₋₆ alkyl, -O-C₁₋₆ alkyl, -C₁₋₆ alkylene-optionally substituted aryl, oxo, or -C(=O)-C₁₋₆ alkyl.
(4-3) The compound or a salt thereof, wherein R^{3a} is H and R^{3b} is H, C₁₋₆ alkyl, -O-C₁₋₆ alkyl, -C₁₋₆ alkylene-optionally substituted aryl, oxo, or -C(=O)-C₁₋₆ alkyl.
(4-4) The compound or a salt thereof, wherein R^{3a} is H and R^{3b} is H or C₁₋₆ alkyl.
(4-5) The compound or a salt thereof, wherein R^{3a} is H and R^{3b} is C₁₋₆ alkyl.
(4-6) The compound or a salt thereof, wherein R^{3a} and R^{3b} are each H.
(5-1) The compound or a salt thereof, wherein R⁴ is C₁₋₆ alkyl substituted with one to three R⁵, C₃₋₈ cycloalkyl optionally substituted with one to four R⁶, 4- to 7-membered saturated heterocyclyl optionally substituted with one to four R⁷, or heteroaryl optionally substituted with one to four R⁸.
(5-2) The compound or a salt thereof, wherein R⁴ is C₁₋₆ alkyl substituted with one R⁵, C₃₋₈ cycloalkyl optionally substituted with one to two R⁶, 4- to 7-membered saturated heterocyclyl optionally substituted with one to two R⁷, or unsubstituted heteroaryl.
(5-3) The compound or a salt thereof, wherein R⁴ is C₃₋₈ cycloalkyl optionally substituted with one to four R⁶ or 4- to 7-membered saturated heterocyclyl optionally substituted with one to four R⁷.
(5-4) The compound or a salt thereof, wherein R⁴ is C₃₋₈ cycloalkyl substituted with one R⁶ or 4- to 7-membered saturated heterocyclyl substituted with one R⁷.
(5-5) The compound or a salt thereof, wherein R⁴ is C₃₋₈ cycloalkyl optionally substituted with one to four R⁶.
(5-6) The compound or a salt thereof, wherein R⁴ is C₃₋₈ cycloalkyl optionally substituted with one to three R⁶.
(5-7) The compound or a salt thereof, wherein R⁴ is C₃₋₈ cycloalkyl optionally substituted with one to two R⁶.
(5-8) The compound or a salt thereof, wherein R⁴ is C₃₋₈ cycloalkyl optionally substituted with one R⁶.
(5-9) The compound or a salt thereof, wherein R⁴ is C₃₋₈ cycloalkyl substituted with one R⁶.
(5-10) The compound or a salt thereof, wherein R⁴ is unsubstituted C₃₋₈ cycloalkyl.
(5-11) The compound or a salt thereof, wherein R⁴ is 4- to 7-membered saturated heterocyclyl optionally substituted with one to four R⁷.
(5-12) The compound or a salt thereof, wherein R⁴ is 4- to 7-membered saturated heterocyclyl optionally substituted with one to three R⁷.
(5-13) The compound or a salt thereof, wherein R⁴ is 4- to 7-membered saturated heterocyclyl optionally substituted with one to two R⁷.
(5-14) The compound or a salt thereof, wherein R⁴ is 4- to 7-membered saturated heterocyclyl optionally substituted with one R⁷.
(5-15) The compound or a salt thereof, wherein R⁴ is 4- to 7-membered saturated heterocyclyl substituted with one R⁷.
(5-16) The compound or a salt thereof, wherein R⁴ is unsubstituted 4- to 7-membered saturated heterocyclyl.
(5-17) The compound or a salt thereof, wherein R⁴ is C₁₋₆ alkyl substituted with one R⁵, C₃₋₈ cycloalkyl optionally substituted with one to two R⁶, or 4- to 7-membered saturated heterocyclyl optionally substituted with one R⁷.
(6-1) The compound or a salt thereof, wherein R⁵ is C₁₋₆ alkyl, C₃₋₈ cycloalkyl, halogeno C₁₋₆ alkyl, 4- to 7-membered saturated heterocyclyl, oxo, cyano, -C(=O)-C₁₋₆ alkyl, -S(=O)₂-C₁₋₆ alkyl, -C(=O)-O-C₁₋₆ alkyl, -C(=O)-NR⁹R¹⁰, -NR⁹R¹⁰, -C₁₋₆ alkylene-OR¹¹, or - OR¹¹.
(6-2) The compound or a salt thereof, wherein R⁵ is -NR⁹R¹⁰ or -OR¹¹.
(6-3) The compound or a salt thereof, wherein R⁵ is -NR⁹R¹⁰.
(6-4) The compound or a salt thereof, wherein R⁵ is -OR¹¹.
(7-1) The compound or a salt thereof, wherein R⁶ is C₁₋₆ alkyl, C₃₋₈ cycloalkyl, halogeno C₁₋₆ alkyl, 4- to 7-membered saturated heterocyclyl, oxo, cyano, -C(=O)-C₁₋₆ alkyl, -S(=O)₂-C₁₋₆ alkyl, -C(=O)-O-C₁₋₆ alkyl, -C(=O)-NR⁹R¹⁰, -NR⁹R¹⁰, -C₁₋₆ alkylene-OR¹¹, or - OR¹¹.
(7-2) The compound or a salt thereof, wherein R⁶ is C₁₋₆ alkyl, cyano, -NR⁹R¹⁰, or -OR¹¹.
(7-3) The compound or a salt thereof, wherein R⁶ is C₁₋₆ alkyl or -OR¹¹.
(7-4) The compound or a salt thereof, wherein R⁶ is -OR¹¹.
(8-1) The compound or a salt thereof, wherein R⁷ is C₁₋₆ alkyl, C₃₋₈ cycloalkyl, halogeno C₁₋₆ alkyl, 4- to 7-membered saturated heterocyclyl, oxo, cyano, -C(=O)-C₁₋₆ alkyl, -S(=O)₂-C₁₋₆ alkyl, -C(=O)-O-C₁₋₆ alkyl, -C(=O)-NR⁹R¹⁰, -NR⁹R¹⁰, -C₁₋₆ alkylene-OR¹¹, or - OR¹¹.
(8-2) The compound or a salt thereof, wherein R⁷ is C₁₋₆ alkyl, C₃₋₈ cycloalkyl, halogeno C₁₋₆ alkyl, 4- to 7-membered saturated heterocyclyl, oxo, -C(=O)-C₁₋₆ alkyl, -S(=O)₂-C₁₋₆ alkyl, - C(=O)-O-C₁₋₆ alkyl, -C(=O)-NR⁹R¹⁰, -C₁₋₆ alkylene-OR¹¹, or -OR¹¹.
(8-3) The compound or a salt thereof, wherein R⁷ is C₁₋₆ alkyl, oxo, or -OR¹¹.
(8-4) The compound or a salt thereof, wherein R⁷ is C₁₋₆ alkyl.
(8-5) The compound or a salt thereof, wherein R⁷ is oxo.
(8-6) The compound or a salt thereof, wherein R⁷ is -OR¹¹.
(9-1) The compound or a salt thereof, wherein R⁸ is C₁₋₆ alkyl, C₃₋₈ cycloalkyl, halogeno C₁₋₆ alkyl, 4- to 7-membered saturated heterocyclyl, oxo, cyano, -C(=O)-C₁₋₆ alkyl, -S(=O)₂-C₁₋₆ alkyl, -C(=O)-O-C₁₋₆ alkyl, -C(=O)-NR⁹R¹⁰, -NR⁹R¹⁰, -C₁₋₆ alkylene-OR¹¹, or - OR¹¹.
(9-2) The compound or a salt thereof, wherein R⁸ is C₁₋₆ alkyl, C₃₋₈ cycloalkyl, halogeno C₁₋₆ alkyl, 4- to 7-membered saturated heterocyclyl, -C₁₋₆ alkylene-OR¹¹, or -OR¹¹.
(9-3) The compound or a salt thereof, wherein R⁸ is 4- to 7-membered saturated heterocyclyl.
(10-1) The compound or a salt thereof, wherein R⁹ and R¹⁰ are each independently H, C₁₋₆ alkyl, or -C(=O)-C₁₋₆ alkyl.
(10-2) The compound or a salt thereof, wherein R⁹ is H and R¹⁰ is H, C₁₋₆ alkyl, or -C(=O)-C₁₋₆ alkyl.
(10-3) The compound or a salt thereof, wherein R⁹ and R¹⁰ are each independently H or C₁₋₆ alkyl.
(10-4) The compound or a salt thereof, wherein R⁹ is H and R¹⁰ is C₁₋₆ alkyl.
(10-5) The compound or a salt thereof, wherein R⁹ and R¹⁰ are each H.
[0056] (11-1) The compound or a salt thereof, wherein R¹¹ is H, C₁₋₆ alkyl, or -C(=O)-C₁₋₆ alkyl.
(11-2) The compound or a salt thereof, wherein R¹¹ is H or C₁₋₆ alkyl.
(11-3) The compound or a salt thereof, wherein R¹¹ is H.
(12) The compound or a salt thereof, which is a combination of two or more groups of the aspects described in (1-1) to (11-3) above that do not contradict each other.
   Examples thereof include, but are not limited to, the following combinations.
(12-1) The compound or a salt thereof, which is a combination of (1-1), (2-1), (3-1), (4-1), (5-1), (6-1), (7-1), (8-1), (9-1), (10-1), and (11-1).
(12-2) The compound or a salt thereof, which is a combination of (1-2), (2-2), (3-1), (4-2), (5-2), (6-2), (7-2), (8-2), (10-2), and (11-1).
(12-3) The compound or a salt thereof, which is a combination of (1-3), (2-2), (3-1), (4-2), (5-3), (7-2), (8 -2), (10-2), and (11-1).
(12-4) The compound or a salt thereof, which is a combination of (1-4), (2-5), (3-3), (4-4), (5-4), (7-4), (8-3), and (11-3).
(12-5) The compound or a salt thereof, which is a combination of (1-5), (2-7), (3-3), (4-6), (5-15), and (8-5).
(12-6) The compound or a salt thereof, which is a combination of (1-5), (2-8), (3-3), (4-6), (5-15), and (8-5).
(12-7) The compound or a salt thereof, which is a combination of (1-2), (2-3), (3-1), (4-2), (5-2), (6-2), (7 (-2), (8-2), (10-2), and (11-1).
(12-8) The compound or a salt thereof, which is a combination of (1-5), (2-5), (3-3), (4-6), (5-15), and (8-5).
(12-9) The compound or a salt thereof, which is a combination of (1-5), (2-9), (3-3), (4-6), (5-17), (6-4), (7-3), (8-5), and (11-3).

Examples of a specific compound included in the present invention include a compound or a salt thereof selected from the group consisting of:
2-{8-[(1R,2R)-2-hydroxycyclohexyl]-4-methyl-5,6,7,8-tetrahydropyrido[2,3-c]pyridazin-3-yl}-5-(trifluoromethyl)phenol;
2-{4-methyl-8-[(3R)-1-methylpiperidin-3-yl]-5,6,7,8-tetrahydropyrido[2,3-c]pyridazin-3-yl }-5-(trifluoromethyl)phenol;
2-{8-[(3R)-1-ethylpiperidin-3-yl]-4-methyl-5,6,7,8-tetrahydropyrido[2,3-c]pyridazin-3-yl}-5-(trifluoromethyl)phenol;
5-(difluoromethoxy)-2-{8-[(1R,2R)-2-hydroxycyclohexyl]-4-methyl-5,6,7,8-tetrahydropyrido[2,3-c]pyridazin-3-yl}phenol;
(3S,4R)-4-{3-[4-(difluoromethoxy)-2-hydroxyphenyl]-4-methyl-6,7-dihydropyrido[2,3-c]pyridazin-8(5H)-yl}oxan-3-ol;
2-{9-[(1R,2R)-2-hydroxycyclohexyl]-4-methyl-5,7,8,9-tetrahydropyridazino[3,4-e][1,4]oxaazepin-3-yl}-5-(trifluoromethyl)phenol;
5-(difluoromethoxy)-2-{9-[(1R,2R)-2-hydroxycyclohexyl]-4-methyl-5,7,8,9-tetrahydropyridazino[3,4-e][1,4]oxaazepin-3-yl}phenol;
(3S)-3-{3-[2-hydroxy-4-(trifluoromethyl)phenyl]-4-methyl-5,6-dihydro-7H-pyrrolo[2,3-c]pyridazin-7-yl}pyrrolidin-2-one;
5-methoxy-2-{4-methyl-9-[(3R)-1-methylpiperidin-3-yl]-5,7,8,9-tetrahydropyridazino[3,4-e][1,4]oxaazepin-3-yl}phenol;
2-{9-[(1R,2R)-2-hydroxycyclohexyl]-4,6-dimethyl-6,7,8,9-tetrahydro-5H-pyridazino[3,4-e][1,4]diazepin-3-yl }-5-(trifluoromethyl)phenol; and
2-{9-[(1R,2R)-2-hydroxycyclohexyl]-4-methyl-6,7,8,9-tetrahydro-5H-pyridazino[3,4-e][1,4]diazepin-3-yl}-5-(trifluoromethyl)phenol.

In addition, examples of a specific compound included in the present invention include a compound or a salt thereof selected from the group consisting of:
2-{8-[(1R,2R)-2-hydroxycyclohexyl]-4-methyl-5,6,7,8-tetrahydropyrido[2,3-c]pyridazin-3-yl}-5-(trifluoromethyl)phenol;
2-{4-methyl-8-[(3R)-1-methylpiperidin-3-yl]-5,6,7,8-tetrahydropyrido[2,3-c]pyridazin-3-yl}-5-(trifluoromethyl)phenol;
2-{8-[(3R)-1-ethylpiperidin-3-yl]-4-methyl-5,6,7,8-tetrahydropyrido[2,3-c]pyridazin-3-yl}-5-(trifluoromethyl)phenol;
5-(difluoromethoxy)-2-{8-[(1R,2R)-2-hydroxycyclohexyl]-4-methyl-5,6,7,8-tetrahydropyrido[2,3-c]pyridazin-3-yl}phenol;
(3S,4R)-4-{3-[4-(difluoromethoxy)-2-hydroxyphenyl]-4-methyl-6,7-dihydropyrido[2,3-c]pyridazin-8(5H)-yl}oxan-3-ol;
2-{9-[(1R,2R)-2-hydroxycyclohexyl]-4-methyl-5,7,8,9-tetrahydropyridazino[3,4-e][1,4]oxaazepin-3-yl}-5-(trifluoromethyl)phenol;
5-(difluoromethoxy)-2-{9-[(1R,2R)-2-hydroxycyclohexyl]-4-methyl-5,7,8,9-tetrahydropyridazino[3,4-e][1,4]oxaazepin-3-yl}phenol;
(35)-3-{3-[2-hydroxy-4-(trifluoromethyl)phenyl]-4-methyl-5,6-dihydro-7H-pyrrolo[2,3-c]pyridazin-7-yl}pyrrolidin-2-one;
5-methoxy-2-{4-methyl-9-[(3R)-1-methylpiperidin-3-yl]-5,7,8,9-tetrahydropyridazino[3,4-e][1,4]oxaazepin-3-yl}phenol;
2-{9-[(1R,2R)-2-hydroxycyclohexyl]-4,6-dimethyl-6,7,8,9-tetrahydro-5H-pyridazino[3,4-e][1,4]diazepin-3-yl}-5-(trifluoromethyl)phenol;
2-{9-[(1R,2R)-2-hydroxycyclohexyl]-4-methyl-6,7,8,9-tetrahydro-5H-pyridazino[3,4-e][1,4]diazepin-3-yl}-5-(trifluoromethyl)phenol; and
(3S)-3-{3-[4-(difluoromethoxy)-2-hydroxyphenyl]-4-methyl-5,6-dihydro-7H-pyrrolo[2,3-c]pyridazin-7-yl}pyrrolidin-2-one.

The compound of formula (I) may have a tautomer or a geometrical isomer depending on the type of the substituent. Although the compound of formula (I) or a salt thereof may be described herein only in one form of an isomer, the present invention also includes isomers other than that as well as separated isomers or mixtures thereof.

In addition, the compound of formula (I) or a salt thereof may have an asymmetric center or an axis chirality in some cases, based on which enantiomers (optical isomers) may exist. The compound of formula (I) or a salt thereof includes any of an isolated individual enantiomer such as (R) form and (S) form, and a mixture thereof (including a racemic mixture or a non-racemic mixture). In an aspect, the enantiomer is "stereochemically pure". The term "stereochemically pure" refers to a degree of purity such that those skilled in the art can recognize an enantiomer as being substantially stereochemically pure. In another aspect, the enantiomer is, for example, a compound having stereochemical purity of 90% ee (enantiomeric excess) or more, 95% ee or more, 98% ee or more, or 99% ee or more.

The present invention further includes a pharmaceutically acceptable prodrug of the compound represented by formula (I). The pharmaceutically acceptable prodrug is a compound having a group that can be converted into, for example, an amino group, a hydroxy group, and a carboxyl group, by solvolysis or under physiological conditions. Examples of the group to form a prodrug include groups described in Prog. Med., 5, 2157-2161 (1985) or in *"*Iyakuhin no Kaihatsu (Pharmaceutical Research and Development)" (Hirokawa Publishing Company, 1990), vol. 7, Bunshi Sekkei (Molecular Design), 163-198.

In addition, the salt of the compound of formula (I) is a pharmaceutically acceptable salt of the compound of formula (I), which may form an acid addition salt or a salt with a base in some cases, depending on the type of the substituent. Specific examples thereof include an acid addition salt of an inorganic acid such as hydrochloric acid, hydrobromic acid, hydroiodic acid, sulfuric acid, nitric acid, and phosphoric acid, or of an organic acid such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid; a salt of an inorganic base such as sodium, potassium, magnesium, calcium, and aluminum, or of an organic base such as methylamine, ethylamine, ethanolamine, lysine, and ornithine; salts of various amino acids and amino acid derivatives such as acetylleucine; and an ammonium salt.

The present invention further includes substances having various hydrates, solvates, and crystalline polymorphism of the compound of formula (I) and a salt thereof.

The present invention also includes all pharmaceutically acceptable compounds of formula (I) or salts thereof, which are labeled with one or more radioactive or nonradioactive isotopes. Examples of suitable isotopes used for isotopic labeling of the compound of the present invention include isotopes such as hydrogen (e.g., ²H and ³H), carbon (e.g., ¹¹C, ¹³C, and ¹⁴C), nitrogen (e.g., ¹³N and ¹⁵N), oxygen (e.g., ¹⁵O, ¹⁷O, and ¹⁸O), fluorine (e.g., ¹⁸F), chlorine (e.g., ³⁶Cl), iodine (e.g., ¹²³I and ¹²⁵I), phosphorus (e.g., ³²P), and sulfur (e.g., ³⁵S). The isotope-labeled compound of the invention of the application can be used for research and the like on tissue distribution of drugs and/or substrates. For example, radioactive isotopes such as tritium (³H) and carbon 14 (¹⁴C) can be used for this purpose from the viewpoint of the ease of labeling and the convenience of detection. Substitution by a heavier isotope, for example, substitution of hydrogen by deuterium (²H), is therapeutically advantageous through the improvement of metabolic stability in some cases (for example, increase *in in vivo* half-life, decrease in required dose, or decrease in interaction between drugs). Substitution by positron emitting isotopes (e.g., ¹¹C, ¹⁸F, ¹⁵O, and ¹³N) can be used in a positron emission tomography (PET) test for testing occupancy of a substrate receptor. The isotope-labeled compound of the present invention can be generally produced by a conventional method known to a person skilled in the art or by the same preparation method as in Examples or Production Examples by using suitable reagents which are labeled with isotopes in place of unlabeled reagents.

### (Preparation Method)

The compound of formula (I) and a salt thereof can be produced by applying various known synthetic methods using the basic structure thereof or the characteristics based on the types of substituents. Depending on the type of a functional group, it is effective for production technique in some cases to replace the functional group with an appropriate protective group (group that can be easily converted to the functional group) in advance at a stage from a raw material to an intermediate. Examples of such a protective group include a protective group described in "Greene's Protective Groups in Organic Synthesis (4th edition, 2006)" by Wuts (P.G.M. Wuts) and Greene (T.W. Greene), and the protective group may be appropriately selected and used according to these reaction conditions. In such a method, a desired compound can be obtained by introducing the protective group to carry out the reaction, then removing the protective group, if necessary.

Similarly to the protective group, a prodrug of the compound of formula (I) can also be produced by introducing a special group at a stage from a raw material to an intermediate or further carrying out the reaction using the obtained compound of formula (I). The reaction can be carried out by applying a method known to a person skilled in the art, such as general esterification, amidation, and dehydration.

Hereinafter, a representative method for producing the compound of formula (I) will be described. Each preparation method can also be applied with reference to reference documents attached to the description. Note that the preparation method of the present invention is not limited to the Examples described below.

In the present specification, the following abbreviations are sometimes used. DMF: N,N-dimethylformamide, DMSO: dimethyl sulfoxide, HPLC: high performance liquid chromatography, DIPEA: N,N-diisopropylethylamine, NMP: 1-methylpyrrolidin-2-one, Me: methyl, Pd-118: [1,1'-bis(di-tert-butylphosphino)ferrocene]dichloropalladium(II), PdCl₂(PPh₃)₂: bis(triphenylphosphine)palladium(II) dichloride, PdCl₂(dppf): [1,1'-bis(diphenylphosphino)ferrocene]Pd(II) dichloride, PdCl₂(dppf)·CH₂Cl₂: [1,1'-bis(diphenylphosphino)ferrocene]Pd(II) dichloride·dichloromethane adduct, Pd₂(dba)₃:(1E,4E)-1,5-diphenylpenta-1,4-dien-3-one/palladium (3:2), Pd(PPh₃)₄: tetrakis(triphenylphosphine) palladium, RuPhos Pd G3: (2-dicyclohexylphosphino-2',6'-diisopropoxy-1,1'-biphenyl)[2-(2'-amino-1,1'-biphenyl)]palladium(II) methanesulfonate, SPhos Pd G3: (2-dicyclohexylphosphino-2',6'-dimethoxybiphenyl)[2-(2'-amino-1,1'-biphenyl)]palladium(II)methanesulfonate, THF: tetrahydrofuran, ODS: octadecylsilyl.

### (First preparation method)

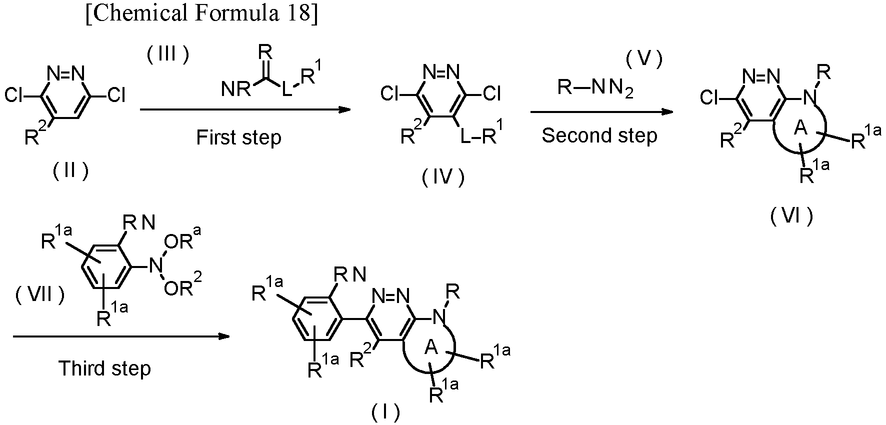

(wherein L is -L¹-L²-L³-L⁴-, L¹ and L⁴ are each CH₂, L² and L³ are each a bond, CH₂, NH, S, or O, the L is substituted with R^{3a} and R^{3b}, X¹ is halogen, methanesulfonyloxy, or p-toluenesulfonyloxy, R^{a} and R^{b} are both H, or R^{a} and R^{b} together with a boronic acid residue attached to the R^{a} and R^{b} form 4,4,5,5-tetramethyl-1,3,2-dioxaborolane; and the same applies to the following).

### (First step)

This step is a step of reacting a compound of formula (II) with a compound of formula (III) to give a compound of formula (IV). In this reaction, the compound of formula (II) and the compound of formula (III) are used in equal amounts or either thereof in an excess amount. A mixture thereof is stirred in the presence of silver nitrate, an acid, and a reoxidizer, in a reaction-inert solvent, under cooling to heating, preferably at 0 to 100°C, usually for 0.1 hours to 5 days. Examples of the solvent used here include, but are not particularly limited to, halogenated hydrocarbons such as dichloromethane and chloroform, acetonitrile, NMP, DMF, DMSO, water, and a mixture thereof. Examples of the acid include, but are not limited to, sulfuric acid and trifluoroacetic acid. Examples of the reoxidizer include, but are not particularly limited to, ammonium peroxodisulfate and potassium peroxodisulfate.

### [Reference]

Angewandte Chemie International Edition, 58, pp 13666-13699 (2019)

### (Second step)

This step is a step of reacting the compound of formula (IV) with a compound of formula (V) to give a compound of formula (VI). In this reaction, the compound of formula (IV) and the compound of formula (V) are used in equal amounts or either thereof in an excess amount. A mixture thereof is stirred in the presence of a base, in a reaction-inert solvent, under cooling to heating, preferably at 0 to 190°C, usually for 0.1 hours to 5 days. Examples of the solvent used here include, but are not particularly limited to, ethers such as diethyl ether, THF, 1,4-dioxane, and 1,2-dimethoxyethane, halogenated hydrocarbons such as dichloromethane and chloroform, pyridine, acetonitrile, NMP, DMF, DMSO, and a mixture thereof. Examples of the base include, but are not particularly limited to, triethylamine and DIPEA. The reaction may also be performed under microwave irradiation.

### (Third step)

This step is a step of reacting the compound of formula (VI) with a compound of formula (VII) to give a compound of formula (I). In this reaction, the compound of formula (VI) and the compound of formula (VII) are used in equal amounts or either thereof in an excess amount. A mixture thereof is stirred in the presence of a catalyst and a base, in a reaction-inert solvent, under cooling to heating under reflux, preferably at room temperature to 150°C, usually for 0.1 hours to 5 days. Examples of the catalyst used here include, but are not particularly limited to, Pd(PPh₃)₄, PdCl₂(PPh₃)₂, PdCl₂(dppf), PdCl₂(dppf)·CH₂Cl₂, Pd₂(dba)₃, RuPhos Pd G3, SPhos Pd G3, and Pd-118. Examples of the base include, but are not particularly limited to, tripotassium phosphate, sodium carbonate, potassium carbonate, cesium carbonate, potassium acetate, sodium hydroxide, and sodium *tert*-butoxide. Examples of the solvent used here include, but are not particularly limited to, ethers such as diethyl ether, THF, 1,4-dioxane, and 1,2-dimethoxyethane, aromatic hydrocarbons such as benzene, toluene, and xylene, water, pyridine, acetonitrile, NMP, DMF, DMSO, and a mixture thereof. The reaction may also be performed under microwave irradiation.

### [Reference]

Journal of the American Chemical Society, 127, pp 4685-4696 (2005)

### (Raw Material Synthesis-1)

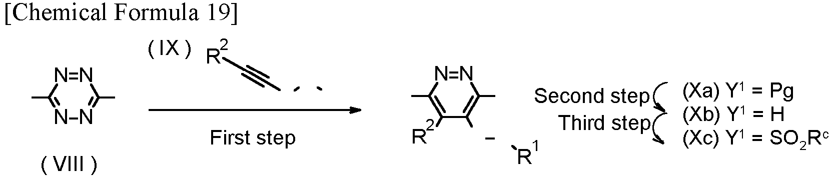

(wherein Pg represents a silicon-containing protective group such as a trimethylsilyl group, a tert-butyl(dimethyl)silyl group, a *tert*-butyl(diphenyl)silyl group; R^{c} represents a methyl group or a p-methylphenyl group; and the same applies to the following).

This preparation method is another method to synthesize a compound (Xc) of synthetic intermediate (IV) where X¹ is a sulfonyloxy group.

### (First step)

This step is a step of reacting a compound of formula (VIII) with a compound of formula (IX) to give a compound of formula (Xa). In this reaction at this step, the compound of formula (VIII) and the compound of formula (IX) are used in equal amounts or either thereof in an excess amount. A mixture thereof is stirred in a reaction-inert solvent, under cooling to heating under reflux, preferably at room temperature to 170°C, usually for 0.1 hours to 5 days. Examples of the solvent used in this reaction include, but are not particularly limited to, aromatic hydrocarbons such as benzene, toluene, xylene, and 1,3,5-trimethylbenzene, carbon halides such as dichloromethane and chloroform, and a mixture thereof.

### (Second step)

This step is a step of deprotecting the compound of formula (Xa) to give a compound of formula (Xb). This step may be performed using the method described in "Greene's Protective Groups in Organic Synthesis (4th edition, 2006)".

### (Third step)

This step is a step of subjecting the compound of formula (Xb) to a sulfonylation reaction to give a compound of formula (Xc). In this reaction at this step, the compound of formula (Xb) and a sulfonylating agent are used in equal amounts or either thereof in an excess amount. A mixture thereof is stirred in a reaction-inert solvent, in the presence of a base, under cooling to heating under reflux, preferably at 0 to 150°C, usually for 0.1 hours to 5 days. Examples of the sulfonylating agent include, but are not particularly limited to, methane sulfonyl chloride and p-toluenesulfonyl chloride. Examples of the solvent used here include, but are not particularly limited to, ethers such as diethyl ether, THF, 1,4-dioxane, and 1,2-dimethoxyethane, halogenated hydrocarbons such as dichloromethane and chloroform, pyridine, acetonitrile, NMP, DMF, DMSO, and a mixture thereof. Examples of the base include, but are not particularly limited to, triethylamine and DIPEA. The addition of 4-(dimethylamino)pyridine may facilitate the reaction.

### (Raw Material Synthesis-2)

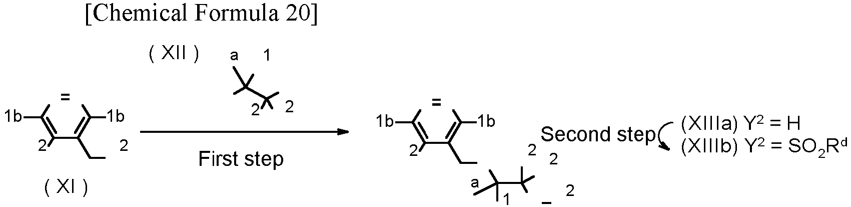

(wherein X² is halogen, methanesulfonyloxy, or p-toluenesulfonyloxy, R^{d} is a methyl group or p-methylphenyl group, Z is NR^{Z} or S, two of R^{e}, R^{f}, R^{g}, R^{h}, and R^{z} are R^{3a}, and R^{3b} and the remaining three are H; and the same applies to the following).

This preparation method is a method of synthesizing a compound (XIIIb) in which L¹ in L of a synthetic intermediate (IV) is CH₂, L² is NH or S, L³ and L⁴ are CH₂, and X¹ is a sulfonyloxy group.

### (First step)

This step is a step of reacting a compound of formula (XI) with a compound of formula (XII) to give a compound of formula (XIIIa). In this reaction at this step, the compound of formula (XI) and the compound of formula (XII) are used in equal amounts or either thereof in an excess amount. A mixture thereof is stirred in a reaction-inert solvent, in the presence of a base, under cooling to heating under reflux, preferably at room temperature to 150°C, usually for 0.1 hours to 5 days. Examples of the solvent used here include, but are not particularly limited to, ethers such as diethyl ether, THF, 1,4-dioxane, and 1,2-dimethoxyethane, halogenated hydrocarbons such as dichloromethane and chloroform, pyridine, acetonitrile, NMP, DMF, DMSO, and a mixture thereof. Examples of the base include, but are not particularly limited to, inorganic bases such as sodium carbonate, potassium carbonate, cesium carbonate, sodium hydroxide, and sodium *tert-*butoxide, and organic bases such as triethylamine and DIPEA.

### (Second step)

This step is a step of obtaining a compound of formula (XIIIb) from the compound of formula (XIIIa). This reaction can be carried out in the same manner as in the third step of Raw Material Synthesis-1 while using a sulfonylating agent.

### (Other Preparation Methods)

The compound of formula (I) obtained by the above preparation method is used as a raw material and further subjected to a chemical modification reaction generally used by those skilled in the art, such as alkylation, benzylation, esterification, amidation, acylation, sulfonylation, carbamylation, carbamoylation, oxidation reaction, reduction reaction, protection reaction, and deprotection reaction to prepare another compound of formula (I).

The compound of formula (I) is isolated and purified as a free compound, a salt thereof, a hydrate, a solvate, or a substance having crystalline polymorphism. The salt of the compound of formula (I) can also be produced by subjecting the compound to a salt formation reaction of a general method. Isolation and purification are carried out by applying usual chemical operations such as extraction, fractional crystallization, and various fractionation chromatography. Various isomers can be produced by selecting an appropriate raw material compound or can be separated by using a difference in physiochemical properties between isomers. For example, an optical isomer can be obtained by a general optical resolution method of racemic form (for example, fractional crystallization leading to a diastereomeric salt with an optically active base or an acid, chromatography using a chiral column or the like), or can also be produced from an appropriate optically active raw material compound.

Pharmacological activity of the compound of formula (I) can be checked by the following tests. Note that the pharmacological activity can also be checked by well-known improved tests.

### Test Example 1: THP-1 IL-1β Production Inhibition Test

PMA (phorbol myristate acetate, SIGMA, P1585) was added to THP-1 cells in an amount of 50 ng/mL to culture the cells for 2 days at 37°C. The culture medium was replaced with a serum-free RPMI-1640 medium, and a compound with a known concentration was added thereto to culture the cells at 37°C for 15 minutes. LPS (Lipopolysaccharide, SIMGA, L2880) and ATP (Adenosine triphosphate, SIGMA, A2383) were added so that final concentrations become 50 ng/mL and 5 mM, respectively, followed by culturing at 37°C for 2 hours. The supernatants were collected to measure the concentrations of IL-1β by ELISA (DuoSet ELISA human IL-1β, R&D Systems, DY201). The IL-1β concentrations were plotted against log concentrations of test compounds, and an IC₅₀ value was calculated by sigmoid Emax model nonlinear regression analysis.

The results are shown in the table below. Example compounds were confirmed to inhibit NLRP3 inflammasome activation and IL-1β production.

**[Table 1]**

| Ex | IC₅₀ (nM) | Ex | IC₅₀ (nM) | Ex | IC₅₀ (nM) | Ex | IC₅₀ (nM) |
|---|---|---|---|---|---|---|---|
| 1 | 16 | 13 | 30 | 26 | 14 | 38 | 7.6 |
| 2 | 6.3 | 14 | 22 | 27 | 42 | 39 | 160 |
| 3 | 3.0 | 15 | 780 | 28 | 8.1 | 40 | 190 |
| 4 | 6.8 | 16 | 1900 | 29 | 13 | 41 | 26 |
| 5 | 13 | 17 | 410 | 30 | 9.5 | 42 | 60 |
| 6 | 14 | 18 | 15 | 31 | 12 | 43 | 230 |
| 7 | 15 | 19 | 30 | 32 | 96 | 44 | 30 |
| 8 | 26 | 20 | 200 | 33 | 55 | 45 | 28 |
| 9 | 35 | 21 | 16 | 34 | 37 | | |
| 10 | 49 | 22 | 120 | 35 | 44 | | |
| 11 | 46 | 23 | 250 | 36 | 11 | | |
| 12 | 44 | 25 | 40 | 37 | 230 | | |

### Test Example 2: Rat Central Nervous System IL-1β Production Test

Under isoflurane anesthesia, 10 to 14 weeks-old male Wistar rats received 12.5 µg/5 µL of LPS (SIGMA, L2880) in the cisterna magna. Two hours later, the test compound, which was suspended in 0.5% methylcellulose solution, was orally administered. After an additional hour, 50 µg/5 µL of BzATP (2'(3')-O-(4-benzoylbenzoyl)adenosine 5'-triphosphate triethylammonium salt, SIGMA, B6396) was administered into the cisterna magna, and cerebrospinal fluid was collected after 30 minutes. The cerebrospinal fluid was subjected to Western blotting using an anti-IL-1β antibody (Millipore, AB1832P) to quantify IL-1β p17, and the percent inhibition relative to the 0.5% methylcellulose solution administration group was calculated. In this test, a plurality of compounds of formula (I) or salts thereof were found to exert an effect of inhibiting IL-1β production in the brain.

### Test Example 3: Evaluation of Motor Function in Mouse α-Synuclein Fibril-Induced Neuroinflammation Model

To male C57BL/6J mice, 8 µg of mouse α-synuclein fibrotic protein (StressMarq Biosciences Inc., SPR-324) is administered at the left striatum. After 13 to 14 weeks, a test compound suspended in a 0.5% methylcellulose solution is orally administered once per day. To a negative control group, a 0.5% methylcellulose solution is administered. After 4 weeks from the start of administration, motor function is evaluated by a hanging wire test. The mice are allowed to catch a wire horizontally stretched. When the mice fall, they are allowed to catch the wire again. This operation is continued for 3 minutes to record the number of falls.

From the above results, the compound of formula (I) or a salt thereof is expected to be used for prevention and/or treatment, etc., of inflammatory diseases and/or neurodegenerative diseases.

A pharmaceutical composition containing one or more compounds of formula (I) or salts thereof as active ingredients can be prepared by using an excipient commonly used in the art, i.e., a pharmaceutical excipient and a pharmaceutical carrier through a method commonly used.

The administration may be either oral administration with tablets, pills, capsules, granules, powders, solutions and the like or parenteral administration with injections (e.g., intraarticular, intravenous, intramuscular), suppositories, eye drops, ophthalmic ointments, transdermal liquids, ointments, transdermal patches, transmucosal solutions, transmucosal patches, and inhalants.

As a solid composition for the oral administration, tablets, powders, granules, and the like are used. In such a solid composition, one or more active ingredients are mixed with at least one inert excipient. The composition may contain an inert additive, such as a lubricant, a disintegrant, a stabilizer and a solubilizing agent according to a conventional method. The tablets, powders, granules, or pills may be coated with wax, a sugar coating, or a film of a gastric or enteric soluble substance, if necessary.

A liquid composition for the oral administration includes a pharmaceutically acceptable emulsion, solution, suspension, syrup, and elixir agent and contains a generally used inert diluent such as purified water or ethanol. The liquid composition may contain, in addition to the inert diluent, a solubilizing agent, a wetting agent, an adjuvant such as a suspending agent, a sweetening agent, a flavoring agent, an aromatic, or a preservative.

An injection for the parenteral administration contains a sterile aqueous or nonaqueous solution, a suspension, or an emulsion. Examples of the aqueous solvent include distilled water for injection or physiological saline. Examples of the nonaqueous solvent include alcohols such as ethanol. Such a composition may further includes an isotonizing agent, a preservative, a wetting agent, an emulsifier, a dispersant, a stabilizer, or a solubilizing aid. These compositions are sterilized, for example, by filtration through a bacteria-retaining filter, incorporation of a microbicide, or irradiation. In addition, they are used to produce a sterile solid composition, and can be used by being dissolved or suspended in sterile water or a sterile injectable solvent before use.

A topical medication includes an ointment, a plaster, a cream, a jelly, a poultice, a spray, a lotion, an eye drop, and an ophthalmic ointment. The topical medication contains a generally used ointment base, lotion base, aqueous or non-aqueous liquid, suspension, and emulsion.

A transmucosal agent such as an inhalant or a transnasal agent is used in a form of solid, liquid, or semisolid, and can be produced according to a conventionally known method. In addition to a known excipient, for example, a pH adjuster, a preservative, a surfactant, a lubricant, a stabilizer, or a thickener may be appropriately added. The administration can be performed using a device for appropriate inhalation or insufflation. For example, using a known device such as a metering and administering inhalation device or an atomizer, the compound may be administered alone or as a powder of a mixture formulated, or as a solution or a suspension in combination with a pharmaceutically acceptable carrier. A dry powder inhaler or the like may be used for a single administration or multiple administrations, and dry powder or powder-containing capsules can be used. Alternatively, a suitable ejection agent, for example, a form of pressurized aerosol spray using suitable gases such as chlorofluoroalkane or carbon dioxide may be employed.

In the case of the common oral administration, a suitable dosage per day is appropriately about 0.001 to 100 mg/kg per body weight, preferably 0.1 to 30 mg/kg, and still more preferably 0.1 to 10 mg/kg, which is given once or two to four times a day. In the case of intravenous administration, a suitable dosage per day is appropriately about 0.0001 to 10 mg/kg per body weight, which is given once to several times a day. In addition, the transmucosal agent is administered at about 0.001 to 100 mg/kg per body weight once to several times a day. The dosage is appropriately decided according to individual cases in consideration of, for example, symptoms, age, and sex.

Depending on an administration route, a dosage form, an administration site, the types of excipients and additives, the pharmaceutical composition of the present invention contains one or more compounds of formula (I) or salts thereof while having active ingredients in a range of 0.01 to 100% by weight, and in an aspect, in a range of 0.01 to 50% by weight.

The compound of formula (I) can be used in combination with various therapeutic or prophylactic agents for diseases in which the compound of formula (I) is considered to exhibit efficacy. The combination use may be simultaneous administration or separate administration in succession, or administered at a desired interval. A simultaneous administration preparation may be a formulated agent or may be separately formulated.

### EXAMPLES

Hereinafter, the preparation methods for the compound of formula (I) will be described in more detail with reference to Examples. Note that the present invention is not limited to compounds described in Examples below. In addition, the preparation method of starting compounds will be described in Production Examples. The preparation method for the compound of formula (I) is not limited to only the preparation methods of specific Examples described below, and the compound of formula (I) may also be produced by using a combination of the preparation methods or a method obvious to a person skilled in the art.

The following abbreviations are sometimes used in Examples, Production Examples, and Tables as described later:
PEx: Production Example No., Ex: Example No., PSyn: Production Example No. where a compound prepared by the same method, Syn: Example No. where a compound prepared by the same method, Str: chemical structural formula, DAT: physicochemical data, CI+: m/z value in mass spectrometry (ionization method CI, [M+H]+ unless otherwise specified), ESI+: m/z value in mass spectrometry (ionization method ESI, [M+H]+ unless otherwise specified), ¹H-NMR (400 MHz, CDCl₃): δ value (ppm) of a signal in ¹H-NMR in CDCl₃, ¹H-NMR (500 MHz, CDCl₃): δ value (ppm) of a signal in ¹H-NMR in CDCl₃, ¹H-NMR (400 MHz, DMSO-d₆): δ value (ppm) of a signal in ¹H-NMR in DMSO-d₆, ¹H-NMR (500 MHz, DMSO-d₆): δ value (ppm) of a signal in ¹H-NMR in DMSO-d₆, ¹H-NMR (500 MHz, CD₃OD): δ value (ppm) of a signal in ¹H-NMR in CD₃OD, ¹H-NMR (400 MHz, CD₃OD): δ value (ppm) of a signal in ¹H-NMR in CD₃OD, J: coupling constant, s: singlet, d: doublet, t: triplet, dd: double doublet, ddd: double double doublet, tt: triple triplet, br: broad (e.g., br s), m: multiplet.

In the Tables described later, hyphen (-) in the columns of PSyn and Syn indicates that the preparation method is described in sentences in Production Examples or Examples.

For the purpose of convenience, the concentration mol/L is expressed as M. For example, a 1 M aqueous sodium hydroxide solution means a 1 mol/L aqueous sodium hydroxide solution.

### Production Example 1

A mixture of 3,6-dichloro-4-(3-chloropropyl)-5-methylpyridazine (400 mg), (3R)-1-ethylpiperidine-3-amine dihydrochloride (321 mg), triethylamine (0.930 mL), and DMSO (4 mL) was heated at 150°C setting for 30 minutes under microwave irradiation. The reaction mixture was admixed with water, and was extracted with ethyl acetate. The organic layer was washed with saturated aqueous sodium chloride, and then dried over anhydrous sodium sulfate. After the solution was concentrated under reduced pressure, the resulting residue was purified by silica gel column chromatography (dichloromethane/methanol) to afford 3-chloro-8-[(3R)-1-ethylpiperidin-3-yl]-4-methyl-5,6,7,8-tetrahydropyrido[2,3-c]pyridazine (130 mg) as an oil.

### Production Example 2

A mixture of 3,6-dichloro-4-(3-chloropropyl)-5-methylpyridazine (1.37 g) and DMSO (6 mL) was admixed with (3R)-1-methylpiperidine-3-amine dihydrochloride (1.61 g) and triethylamine (4.8 mL) under argon atmosphere. The mixture was then stirred at 150°C for 1 hour under microwave irradiation. The reaction mixture was admixed with water, and was extracted with ethyl acetate. The organic layer was washed with saturated aqueous sodium chloride, and then dried over anhydrous sodium sulfate. After the solution was concentrated under reduced pressure, the resulting residue was purified by silica gel column chromatography (chloroform/methanol) to afford 3-chloro-4-methyl-8-[(3R)-1-methylpiperidin-3-yl]-5,6,7,8-tetrahydropyrido[2,3-c]pyridazine (507 mg) as a solid.

### Production Example 3

A mixture of 3,6-dichloro-4-(3-chloropropyl)-5-methylpyridazine (100 mg), (1R,2R)-2-aminocyclohexan-1-ol (73 mg), triethylamine (0.18 mL), and DMSO (1 mL) was stirred at 150°C for 1 hour under microwave irradiation. The resulting mixture was cooled to room temperature, ethyl acetate and water were added, and the mixture was extracted with ethyl acetate. The organic layer was washed with saturated aqueous sodium chloride, and then dried over anhydrous magnesium sulfate. After the solution was concentrated under reduced pressure, the resulting residue was purified by silica gel column chromatography (hexane/ethyl acetate) to afford (1R,2R)-2-(3-chloro-4-methyl-6,7-dihydropyrido[2,3-c]pyridazin-8(5H)-yl)cyclohexan-1-ol (80 mg) as a solid.

### Production Example 4

A mixture of (3S,4R)-4-aminooxan-3-ol monohydrochloride (488 mg) and DMSO (5 mL) was admixed with triethylamine (1.31 mL) at room temperature under argon atmosphere, and was then stirred at the same temperature for 5 minutes. The resulting mixture was admixed with 3,6-dichloro-4-(3-chloropropyl)-5-methylpyridazine (500 mg) at room temperature, and was then stirred at 150°C for 1 hour under microwave irradiation. The resulting mixture was cooled to room temperature, ethyl acetate and water were added, and the mixture was extracted with ethyl acetate. The organic layer was washed with saturated aqueous sodium chloride, and then dried over anhydrous magnesium sulfate. After the solution was concentrated under reduced pressure, the resulting residue was purified by silica gel column chromatography (hexane/ethyl acetate) to afford (3R,4R)-4-(3-chloro-4-methyl-6,7-dihydropyrido[2,3-c]pyridazin-8(5H)-yl)oxan-3-ol (325 mg) as a solid.

### Production Example 5

Under argon atmosphere, a mixture of 3,6-dichloro-4-[(2-chloroethoxy)methyl]-5-methylpyridazine (200 mg), (1R,2R)-2-aminocyclohexan-1-ol (135 mg), triethylamine (0.220 mL), and DMSO (2 mL) was stirred at 150°C for 2 hours under microwave irradiation. The resulting mixture was admixed with water, and was extracted with ethyl acetate. The organic layer was washed with saturated aqueous sodium chloride, and then dried over anhydrous magnesium sulfate. After the solution was concentrated under reduced pressure, the resulting residue was purified by silica gel column chromatography (chloroform/methanol) to afford (1R,2R)-2-(3-chloro-4-methyl-7,8-dihydropyridazino[3,4-e][1,4]oxaazepin-9(5H)-yl)cyclohexan-1-ol (92.4 mg) as an oil.

### Production Example 6

A mixture of 4-methylbenzene-1-sulfonic acid 2-(3,6-dichloro-5-methylpyridazin-4-yl)ethyl (300 mg) and NMP (1.5 mL) was admixed with (3S)-3-aminopyrrolidin-2-one (126 mg) and triethylamine (0.360 mL) at room temperature. The mixture was then stirred at 100°C for 6 hours. The resulting mixture was cooled to room temperature, ethyl acetate and saturated aqueous sodium chloride/water (1/1) were added, and the aqueous layer was extracted sequentially with ethyl acetate and chloroform/methanol (9/1). The organic layer was washed with saturated aqueous sodium chloride, and then dried over anhydrous sodium sulfate. After the solution was concentrated under reduced pressure, the resulting residue was purified by silica gel column chromatography (chloroform/methanol) to afford (3S)-3-(3-chloro-4-methyl-5,6-dihydro-7H-pyrrolo[2,3-c]pyridazin-7-yl)pyrrolidin-2-one (197 mg) as a solid.

### Production Example 7

A mixture of 3,6-dichloro-4-[(2-chloroethoxy)methyl]-5-methylpyridazine (630 mg) and DMSO (12.6 mL) was admixed with (3R)-1-methylpiperidine-3-amine dihydrochloride (692 mg) and triethylamine (2.06 mL) at room temperature, and was then stirred at 150°C for 3 hours under microwave irradiation. The resulting mixture was admixed with ethyl acetate and saturated aqueous sodium chloride/water (1/1), and the aqueous layer was extracted with ethyl acetate. The combined organic layer was washed with saturated aqueous sodium chloride/water (1/1), and then dried over anhydrous sodium sulfate. After the solution was concentrated under reduced pressure, the resulting residue was purified by silica gel column chromatography (chloroform/methanol) to afford 3-chloro-4-methyl-9-[(3R)-1-methylpiperidin-3-yl]-5,7,8,9-tetrahydropyridazino[3,4-e][1,4]oxaazepine (35 mg) as a solid.

### Production Example 8

A mixture of 2-chloro-N-[(3,6-dichloro-5-methylpyridazin-4-yl)methyl]-N-(methyl)ethan-1-amine (0.209 g), (1R,2R)-2-aminocyclohexan-1-ol (0.136 g), triethylamine (0.43 mL), and DMSO (0.8 mL) was stirred at 150°C for 1 hour under microwave irradiation. The reaction mixture was allowed to cool to room temperature, and was then poured into water. The aqueous layer was extracted with ethyl acetate. The organic layer was washed sequentially with water and saturated aqueous sodium chloride, and then dried over anhydrous sodium sulfate. After the organic layer was concentrated under reduced pressure, the resulting residue was purified by silica gel column chromatography (dichloromethane/methanol) to afford (1R,2R)-2-(3-chloro-4,6-dimethyl-5,6,7,8-tetrahydro-9H-pyridazino[3,4-e][1,4]diazepin-9-yl) cyclohexane-1-ol (0.155 g) as an oil.

### Production Example 9

A mixture of 2-{[(3,6-dichloro-5-methylpyridazin-4-yl)methyl][(4-methoxyphenyl)methyl]amino}ethyl methanesulfonate (1.746 g), (1R,2R)-2-aminocyclohexan-1-ol (0.641 g), triethylamine (2.1 mL), and DMSO (5 mL) was stirred at 150°C for 1.5 hours under microwave irradiation. The reaction mixture was allowed to cool to room temperature, and water was then added. The aqueous layer was extracted with ethyl acetate. The organic layer was washed sequentially with water and saturated aqueous sodium chloride, and then dried over anhydrous sodium sulfate. After the organic layer was concentrated under reduced pressure, the resulting residue was purified by silica gel column chromatography (hexane/ethyl acetate) to afford (1R,2R)-2-{3-chloro-6-[(4-methoxyphenyl)methyl]-4-methyl-5,6,7,8-tetrahydro-9H-pyridazino[3,4-e][1,4]diazepin-9-yl}cyclohexan-1-ol (0.777 g) as a solid.

### Production Example 26

A mixture of 3-chloro-4-methyl-8-[(3R)-piperidin-3-yl]-5,6,7,8-tetrahydropyrido[2,3-c]pyridazine dihydrochloride (500 mg) and acetonitrile (10 mL) was admixed with potassium carbonate (1.04 g) and 2-bromoethanol (0.200 mL) at 25°C, and was then stirred at 80°C for 6 hours. After the resulting mixture was concentrated under reduced pressure, water and ethyl acetate were added to the resulting residue. The mixture was then extracted with ethyl acetate. The organic layer was dried over anhydrous sodium sulfate. After the solution was concentrated under reduced pressure, the resulting residue was purified by silica gel column chromatography (petroleum ether/ethyl acetate) to afford 2-[(3R)-3-(3-chloro-4-methyl-6,7-dihydropyrido[2,3-c]pyridazin-8(5H)-yl)piperidin-1-yl]ethan-1-ol (150 mg) as a solid.

### Production Example 27

A mixture of (3R)-3-(3-chloro-4-methyl-6,7-dihydropyrido[2,3-c]pyridazin-8(5H)-yl)piperidine-1-tert-butyl carboxylic acid (1.5 g) and hydrogen chloride methanol solution (4 M, 10 mL) was stirred at 25°C for 12 hours. The resulting mixture was concentrated under reduced pressure to afford 3-chloro-4-methyl-8-[(3R)-piperidin-3-yl]-5,6,7,8-tetrahydropyrido[2,3-c]pyridazine dihydrochloride (1.5 g) as an oil.

### Production Example 30

A mixture of 3-chloro-4-methyl-8-[(3R)-piperidin-3-yl]-5,6,7,8-tetrahydropyrido[2,3-c]pyridazine dihydrochloride (1.5 g) and 1,2-dichloroethane (10 mL) was admixed with acetic acid (338 mg), and was then stirred at 20°C for 5 minutes. The resulting mixture was admixed with oxetan-3-one (446 mg), sodium triacetoxyborohydride (1.79 g), and triethylamine (0.861 mL), and was then stirred at 40°C for 12 hours. The reaction mixture was admixed with water and ethyl acetate, and was extracted with ethyl acetate. The organic layer was washed with saturated aqueous sodium chloride, and then dried over anhydrous sodium sulfate. After the solution was concentrated under reduced pressure, the resulting residue was purified by silica gel column chromatography (petroleum ether/ethyl acetate) to afford 3-chloro-4-methyl-8-[(3R)-1-(oxetan-3-yl)piperidin-3-yl]-5,6,7,8-tetrahydropyrido[2,3-c]pyridazine (230 mg) as an oil.

### Production Example 32

A mixture of 3,6-dichloro-4-methylpyridazine (2.99 g) and water (60 mL) was admixed with sulfuric acid (4.9 mL) under ice cold conditions over about 10 minutes. The resulting mixture was admixed with 4-chlorobutanoic acid (2.02 mL) and silver nitrate (3.12 g) under ice-cold conditions, followed by stirring for 15 minutes in a water bath. The resulting mixture was admixed with a mixture of ammonium peroxodisulfate (10.5 g) and water (30 mL) at room temperature, and was then stirred at 60°C for 1.5 hours. The reaction mixture was admixed with a mixture of 4-chlorobutanoic acid (0.368 mL), ammonium peroxodisulfate (2.1 g), and water (3 mL) at 70°C, and was then stirred at the same temperature for 40 minutes. The resulting mixture was admixed with a mixture of 4-chlorobutanoic acid (0.368 mL), ammonium peroxodisulfate (2.1 g), and water (3 mL) at 70°C, and was then stirred at the same temperature for 1.5 hours. The reaction mixture was allowed to cool to room temperature, and admixed with 28% aqueous ammonia solution under ice-cold conditions to bring the pH to about 10. The resulting mixture was extracted with ethyl acetate. The organic layer was washed with saturated aqueous sodium chloride, and then dried over anhydrous sodium sulfate. After the solution was concentrated under reduced pressure, the resulting residue was purified by silica gel column chromatography (hexane/ethyl acetate) to afford 3,6-dichloro-4-(3-chloropropyl)-5-methylpyridazine (1.51 g) as an oil.

### Production Example 33

A mixture of 3,6-dichloro-4-methylpyridazine (1 g) and water (30 mL) was admixed with sulfuric acid (1.6 mL) under ice cold conditions. The resulting mixture was admixed with (2-chloroethoxy)acetic acid (980 mg) and silver nitrate (210 mg) under ice cold conditions, and was then stirred for 5 minutes. The resulting mixture was admixed with a mixture of ammonium peroxodisulfate (2.8 g) and water (10 mL) at room temperature, and was then stirred at 70°C for 3 hours. The resulting reaction mixture was admixed with a mixture of (2-chloroethoxy)acetic acid (470 mg), ammonium peroxodisulfate (700 mg), and water (2 mL), and was then stirred at 70°C for 2 hours. The resulting mixture was admixed with a mixture of (2-chloroethoxy)acetic acid (470 mg), ammonium peroxodisulfate (700 mg), and water (2 mL), and was then stirred at 70°C for 11 hours. The resulting mixture was admixed with 28% aqueous ammonia solution (4 mL) under ice-cold conditions and extracted with ethyl acetate. The organic layer was washed with saturated aqueous sodium chloride, and then dried over anhydrous magnesium sulfate. After the solution was concentrated under reduced pressure, the resulting residue was purified by silica gel column chromatography (hexane/ethyl acetate) to afford 3,6-dichloro-4-[(2-chloroethoxy)methyl]-5-methylpyridazine (465 mg) as a solid.

### Production Example 35

To a mixture of potassium carbonate (1.65 g) and DMF (7.4 mL) was added dropwise a mixture of 4-bromo-3-(methoxymethoxy)phenol (1.86 g), sodium chlorodi(fluoro)acetate (2.43 g), and DMF (11 mL) at 95°C over 1 hour, and the resulting mixture was then stirred at the same temperature for 15 minutes. The reaction mixture was cooled to room temperature, and then admixed with potassium carbonate (1.65 g) and sodium chlorodi(fluoro)acetate (2.43 g). The mixture was then stirred at 95°C for 1 hour. The reaction mixture was cooled to room temperature, and then admixed with ethyl acetate and water. The organic layer was separated. The aqueous layer was extracted with ethyl acetate and the combined organic layer was washed with water and saturated aqueous sodium chloride, and then dried over anhydrous sodium sulfate. The solution was concentrated under reduced pressure and the resulting residue was purified by silica gel column chromatography (hexane/ethyl acetate) to afford 1-bromo-4-(difluoromethoxy)-2-(methoxymethoxy)benzene (920 mg) as an oil.

### Production Example 36

A mixture of 1-bromo-4-(difluoromethoxy)-2-(methoxymethoxy)benzene (920 mg) and 1,4-dioxane (9.2 mL) was admixed with 4,4,4',4',5,5,5',5'-octamethyl-2,2'-bi-1,3,2-dioxaborolane (1.24 g), PdCl₂(dppf) (238 mg), and potassium acetate (957 mg), and was then stirred at 100°C for 3 hours. After the reaction mixture was cooled to room temperature, PdCl₂(dppf) (238 mg) was added and the mixture was stirred at 100°C for 2 hours. The reaction mixture was cooled to room temperature, and then admixed with 4,4,4',4',5,5,5',5'-octamethyl-2,2'-bi-1,3,2-dioxaborolane (620 mg). The mixture was stirred at 100°C for 3 hours. The reaction mixture was cooled to room temperature, and then admixed with hexane. The mixture was stirred at room temperature for 30 minutes. The reaction mixture was filtered through Celite (registered trademark) and the filtrate was concentrated under reduced pressure to afford 2-[4-(difluoromethoxy)-2-(methoxymethoxy)phenyl]-4,4,5,5-tetramethyl-1,3,2-dioxaborolane (1.98 g) as an oil.

### Production Example 38

Under argon atmosphere, a mixture of (1R,2R)-2-(3-chloro-4-methyl-7,8-dihydropyridazino[3,4-e][1,4]oxaazepin-9(5H)-yl)cyclohexane-1-ol (79 mg), 2-[4-(difluoromethoxy)-2-(methoxymethoxy)phenyl]-4,4,5,5-tetramethyl-1,3,2-dioxaborolane (150 mg), RuPhos Pd G3 (35 mg), potassium carbonate (88 mg), 1,4-dioxane (1.6 mL), and water (0.4 mL) was stirred at 100°C for 1.5 hours under microwave irradiation. The mixture was allowed to cool to room temperature, and then admixed with water and ethyl acetate. The mixture was extracted with ethyl acetate. The combined organic layer was washed with saturated aqueous sodium chloride, and then dried over anhydrous magnesium sulfate. After the solution was concentrated under reduced pressure, the resulting residue was purified by silica gel column chromatography (chloroform/methanol) to afford (1R,2R)-2-{3-[4-(difluoromethoxy)-2-(methoxymethoxy)phenyl]-4-methyl-7,8-dihydropyridazino[3,4-e][1,4]oxaazepin-9(5H)-yl}cyclohexan-1-ol (113 mg) as a solid.

### Production Example 39

Under argon atmosphere, a mixture of (1R,2R)-2-{3-chloro-6-[(4-methoxyphenyl)methyl]-4-methyl-5,6,7,8-tetrahydro-9H-pyridazino[3,4-e][1,4]diazepin-9-yl}cyclohexane-1-ol (0.762 g), [2-hydroxy-4-(trifluoromethyl)phenyl]boronic acid (0.754 g), PdCl₂(dppf)-CH₂Cl₂ (0.149 g), potassium carbonate (0.506 g), 1,4-dioxane (18 mL), and water (5 mL) was stirred at 90 to 100°C for 24 hours. The reaction mixture was allowed to cool to room temperature, and was then poured into water. The aqueous layer was extracted with ethyl acetate. The organic layer was washed with saturated aqueous sodium chloride, and then dried over anhydrous sodium sulfate. After the organic layer was concentrated under reduced pressure, the resulting residue was purified by silica gel column chromatography (hexane/ethyl acetate) to afford 2-{9-[(1R,2R)-2-hydroxycyclohexyl]-6-[(4-methoxyphenyl)methyl]-4-methyl-6,7,8,9-tetrahydro-5H-pyridazino[3,4-e][1,4]diazepin-3-yl}-5-(trifluoromethyl)phenol (0.700 g) as a solid.

### Production Example 45

A mixture of (3R)-3-{3-[2-hydroxy-4-(trifluoromethyl)phenyl]-4-methyl-6,7-dihydropyrido[2,3-c]pyridazin-8(5H)-yl}piperidine-1-*tert*-butyl carboxylic acid (7.4 g) and dichloromethane (100 mL) was admixed with trifluoroacetic acid (103 mL) at 25°C, and was then stirred at the same temperature for 16 hours. The reaction mixture was concentrated under reduced pressure. The resulting residue was admixed with water, and was washed with ethyl acetate. Saturated sodium bicarbonate solution was added to the aqueous layer to bring the pH to 8, and the mixture was then extracted with ethyl acetate. The organic layer was washed with saturated aqueous sodium chloride, and then dried over anhydrous sodium sulfate. The solution was concentrated under reduced pressure to afford 2-{4-methyl-8-[(3R)-piperidin-3-yl]-5,6,7,8-tetrahydropyrido[2,3-c]pyridazin-3-yl}-5-(trifluoromethyl)phenol (4.1 g) as a solid.

### Production Example 46

A mixture of [(1R,2R)-2-(3-chloro-4-methyl-6,7-dihydropyrido[2,3-c]pyridazin-8(5H)-yl)cyclohexyl]tert-butyl carbamate (600 mg) and DMF (10 mL) was admixed with sodium hydride (94.5 mg, 60% dispersion in mineral oil) at 0°C under a nitrogen atmosphere, and was then stirred at 25°C for 0.5 hours. To the resulting mixture was added methyl iodide (0.147 mL) at 25°C and stirred at the same temperature for 2 hours. The reaction mixture was admixed with water, and was extracted with ethyl acetate. The organic layer was washed with saturated aqueous sodium chloride, and then dried over anhydrous sodium sulfate. After the solution was concentrated under reduced pressure, the resulting residue was purified by silica gel column chromatography (petroleum ether/ethyl acetate) to afford [(1R,2R)-2-(3-chloro-4-methyl-6,7-dihydropyrido[2,3-c]pyridazin-8(5H)-yl)cyclohexyl]tert-butyl(methyl)carbamate (200 mg) as an oil.

### Production Example 47

A mixture of (1R,2R)-2-(3-chloro-4-methyl-6,7-dihydropyrido[2,3-c]pyridazin-8(5H)-yl)cyclohexane-1-amine (860 mg), di-tert-butyl bicarbonate (802 mg) and dichloromethane (10 mL) was admixed with triethylamine (0.512 mL) at 25°C, and was then stirred at the same temperature for 16 hours. The reaction mixture was admixed with water and then extracted with dichloromethane. The organic layer was washed with saturated aqueous sodium chloride, and then dried over anhydrous sodium sulfate. After the solution was concentrated under reduced pressure, the resulting residue was purified by silica gel column chromatography (dichloromethane/methanol) to afford [(1R,2R)-2-(3-chloro-4-methyl-6,7-dihydropyrido[2,3-c]pyridazin-8(5H)-yl)cyclohexyl]tert-butyl carbamate (700 mg) as an oil.

### Production Example 48

A mixture of 2-(3,6-dichloro-5-methylpyridazin-4-yl)ethan-1-ol (500 mg) and dichloromethane (5 mL) was admixed with triethylamine (0.840 mL), p-toluenesulfonyl chloride (920 mg), 4-(dimethylamino)pyridine (15 mg) under ice cold conditions. The mixture was stirred at the same temperature for 2 hours. The reaction mixture was admixed with dichloromethane and ice, and was stirred at room temperature for 30 minutes. The resulting mixture was admixed with saturated aqueous sodium chloride/water (1/1), and the organic layer was then dried over anhydrous sodium sulfate. After the solution was concentrated under reduced pressure, the resulting residue was purified by silica gel column chromatography (hexane/ethyl acetate) to afford 2-(3,6-dichloro-5-methylpyridazin-4-yl)ethyl 4-methylbenzen-1-sulfonate (812 mg) as a solid.

### Production Example 50

A mixture of 4-(2-{[*tert*-butyl di(phenyl)silyl]oxy}ethyl)-3,6-dichloro-5-methylpyridazine (14.6 g) and THF (290 mL) was admixed with tetrabutylammonium fluoride THF solution (1 M, 49 mL) under ice cold conditions, and then stirred at the same temperature for 0.5 hours. The reaction mixture was admixed with water under ice cold conditions, and was extracted with ethyl acetate. The organic layer was washed sequentially with water and saturated aqueous sodium chloride, and then dried over anhydrous sodium sulfate. After the solution was concentrated under reduced pressure, the resulting residue was purified by silica gel column chromatography (hexane/ethyl acetate) to afford 2-(3,6-dichloro-5-methylpyridazin-4-yl)ethan-1-ol (5.55 g) as an oil.

### Production Example 52

A mixture of 3,6-dichloro-1,2,4,5-tetrazine (450 mg), *tert*-butyl[(penta-3-yn-1-yl)oxy]di(phenyl)silane (1.15 g), and 1,3,5-trimethylbenzene (3 mL) was stirred at 160°C for 16 hours. The mixture was cooled to room temperature, and was then concentrated under reduced pressure. The resulting residue was purified by silica gel column chromatography (hexane/ethyl acetate) to afford 4-(2-{[*tert*-butyl di(phenyl)silyl]oxy}ethyl)-3,6-dichloro-5-methylpyridazine (1.2 g) as an oil.

### Production Example 54

A mixture of 2-{[(3,6-dichloro-5-methylpyridazin-4-yl)methyl][(4-methoxyphenyl)methyl]amino}ethan-1-ol (1.322 g) and dichloromethane (37 mL) was admixed with methane sulfonyl chloride (0.35 mL) and triethylamine (1.1 mL) with stirring under ice cold conditions, and was then stirred for 3.5 hours while the temperature was allowed to rise spontaneously to room temperature. Saturated ammonium chloride solution was added to the reaction mixture, and the aqueous layer was extracted with dichloromethane. The organic layer was dried over anhydrous sodium sulfate and the organic layer was concentrated under reduced pressure to afford 2-{[(3,6-dichloro-5-methylpyridazin-4-yl)methyl][(4-methoxyphenyl)methyl]amino}ethyl methanesulfonate (1.776 g) as an oil.

### Production Example 55

A mixture of 2-(3,6-dichloro-5-methylpyridazin-4-yl)ethan-1-ol (0.96 g) and ethyl acetate (10 mL) was admixed with triethylamine (0.65 mL) and methanesulfonyl chloride (0.36 mL) under ice cold conditions, and was then stirred at the same temperature for 10 minutes. After the insoluble material was filtered off, the filtrate was concentrated under reduced pressure to afford 2-(3,6-dichloro-5-methylpyridazin-4-yl)ethyl methanesulfonate (1.4 g) as an oil.

### Production Example 56

A mixture of 3-(3,6-dichloro-5-methylpyridazin-4-yl)-3-methoxypropan-1-ol (30 mg), triethylamine (0.018 mL), and dichloromethane (1 mL) was admixed with methane sulfonic anhydride (25 mg) at 0°C, and was then stirred at the same temperature for 30 minutes. The reaction mixture was concentrated under reduced pressure to afford 3-(3,6-dichloro-5-methylpyridazin-4-yl)-3-methoxypropyl methanesulfonate (39 mg) as an oil. The product was used in the next reaction without purification.

### Production Example 57

Under argon atmosphere, a mixture of 2-{[(3,6-dichloro-5-methylpyridazin-4-yl)methyl]sulfanyl}ethan-1-ol (0.671 g) and dichloromethane (27 mL) was admixed with triethylamine (0.81 mL) and methane sulfonyl chloride (0.25 mL) with stirring under ice-cold conditions. The mixture was stirred for 2.5 hours while the temperature was allowed to rise spontaneously to room temperature. Saturated ammonium chloride solution was added to the reaction mixture, and the aqueous layer was extracted with dichloromethane and the organic layer was washed with saturated aqueous ammonium chloride. The organic layer was dried over anhydrous sodium sulfate and the organic layer was concentrated under reduced pressure to afford 2-{[(3,6-dichloro-5-methylpyridazin-4-yl)methyl]sulfanyl}ethyl methanesulfonate(0.770 g) as an oil.

### Production Example 58

A mixture of (2R)-2-{[(3,6-dichloro-5-methylpyridazin-4-yl)methyl][(4-methoxyphenyl)methyl]amino}propan-1-ol (1.705 g) and dichloromethane (46 mL) was admixed with methansulfonyl chloride (0.43 mL) and triethylamine (1.4 mL) with stirring under ice cold conditions, and was then stirred for 3 hours while the temperature was allowed to rise spontaneously to room temperature. Saturated ammonium chloride solution was added to the reaction mixture, and the aqueous layer was extracted with dichloromethane. The organic layer was dried over anhydrous sodium sulfate and the organic layer was concentrated under reduced pressure to afford (2R)-2-{[(3,6-dichloro-5-methylpyridazin-4-yl)methyl][(4-methoxyphenyl)methyl]amino} propyl methanesulfonate (2.233 g) as an oil.

### Production Example 59

A mixture of *tert*-butyl[(3-methoxypenta-4-yn-1-yl)oxy]di(phenyl)silane (100 mg) and THF (2 mL) was admixed with n-butyl lithium (2.5 M, 0.227 mL) at -78°C under nitrogen atmosphere, and was then stirred at the same temperature for 30 minutes. The resulting mixture was admixed with methyl iodide (0.018 mL), and was stirred at 15°C for 90 minutes. The reaction mixture was admixed with water, and was then extracted with ethyl acetate. The organic layer was dried over anhydrous sodium sulfate. After the solution was concentrated under reduced pressure, the resulting residue was purified by silica gel column chromatography (petroleum ether/ethyl acetate) to afford *tert*-butyl[(3-methoxyhexa-4-yn-1-yl)oxy]di(phenyl)silane (80 mg) as an oil.

### Production Example 60

A mixture of 3-methoxypenta-4-yn-1-ol (700 mg) and dichloromethane (10 mL) was admixed with imidazole (626 mg) and *tert*-butyl(chloro)di(phenyl)silane (1.73 mL), and was then stirred at 15°C for 12 hours. The reaction mixture was admixed with water and then extracted with dichloromethane. The organic layer was dried over anhydrous sodium sulfate. After the solution was concentrated under reduced pressure, the resulting residue was purified by silica gel column chromatography (petroleum ether/ethyl acetate) to afford *tert*-butyl[(3-methoxypenta-4-yn-1-yl)oxy]di(phenyl)silane (630 mg) as an oil.

### Production Example 61

A mixture of [(3-{[(1R,2R)-2-hydroxycyclohexyl]amino}-6-[2-hydroxy-4-(trifluoromethyl)phenyl]-5-methylpyridazin-4-yl)methyl]*tert*-butyl carbamate (0.403 g), trifluoroacetic acid (4 mL), and dichloromethane (8 mL) was stirred at room temperature for 24 hours. Saturated sodium bicarbonate solution was added to the reaction mixture, and the aqueous layer was then extracted with ethyl acetate. The organic layer was washed with saturated aqueous sodium chloride, and then dried over anhydrous sodium sulfate. After the organic layer was concentrated under reduced pressure, the resulting residue was purified by silica gel column chromatography (dichloromethane/methanol) to afford 2-[5-(aminomethyl)-6-{[(1R,2R)-2-hydroxycyclohexyl]amino}-4-methylpyridazin-3-yl]-5-(trifluoromethyl)phenol (0.242 g) as a solid.

### Production Example 62

A mixture of [(3,6-dichloro-5-methylpyridazin-4-yl)methyl]*tert*-butyl carbamate (1.608 g), (1R,2R)-2-aminocyclohexan-1-ol (1.261 g), DIPEA (2.3 mL), and cyclopentanol (5.5 mL) was stirred at 150 to 155°C for 19 hours. The reaction mixture was admixed with water and ethyl acetate, and the aqueous layer was extracted with ethyl acetate. The organic layer was washed with saturated aqueous sodium chloride, and then dried over anhydrous sodium sulfate. The organic layer was concentrated under reduced pressure. The resulting residue was purified by silica gel column chromatography (hexane/ethyl acetate) to afford [(6-chloro-3-{[(1R,2R)-2-hydroxycyclohexyl]amino}-5-methylpyridazin-4-yl)methyl]*tert-*butyl carbamate (0.824 g) as a solid.

### Production Example 63

A mixture of 1-(3,6-dichloro-5-methylpyridazin-4-yl)methaneamine monohydrochloride (0.615 g), di-*tert*-butyl bicarbonate (0.93 mL), triethylamine (1.13 mL), and dichloromethane (9 mL) was stirred at 0°C for 1 hour and then at room temperature for 19 hours. After the reaction mixture was concentrated under reduced pressure, water and ethyl acetate were added to the resulting residue. The aqueous layer was then extracted with ethyl acetate. The organic layer was washed with saturated aqueous sodium chloride, and then dried over anhydrous sodium sulfate. The organic layer was concentrated under reduced pressure to afford [(3,6-dichloro-5-methylpyridazin-4-yl)methyl]*tert*-butyl carbamate (0.949 g) as an oil.

### Production Example 64

A mixture of N-[(3,6-dichloro-5-methylpyridazin-4-yl)methyl]-1,1,1-triphenylmethanamine (1.400 g), hydrogen chloride 1,4-dioxane solution (4 M, 4 mL), and dichloromethane (8 mL) was stirred at room temperature for 15 hours. The precipitated solid was filtered off and washed with diethyl ether to afford 1-(3,6-dichloro-5-methylpyridazin-4-yl)methaneamine monohydrochloride (0.615 g) as a solid.

### Production Example 65

A mixture of 4-(bromomethyl)-3,6-dichloro-5-methylpyridazine (1.003 g), 2-(methylamino)ethan-1-ol (0.47 mL), potassium carbonate (1.631 g), and acetonitrile (20 mL) was stirred at 51°C for 5 hours. The reaction mixture was poured into water. The aqueous layer was then extracted with ethyl acetate. The organic layer was washed with saturated aqueous sodium chloride, and then dried over anhydrous sodium sulfate. After the organic layer was concentrated under reduced pressure, the resulting residue was purified by silica gel column chromatography (hexane/ethyl acetate) to afford 2-{[(3,6-dichloro-5-methylpyridazin-4-yl)methyl](methyl)amino}ethan-1-ol (0.626 g) as an oil.

### Production Example 66

A mixture of 4-(bromomethyl)-3,6-dichloro-5-methylpyridazine (2.035 g), 2-{[(4-methoxyphenyl)methyl]amino}ethan-1-ol (1.441 g), potassium carbonate (2.198 g), and acetonitrile (40 mL) was stirred at 50°C for 5 hours. The reaction mixture was allowed to cool to room temperature, and water was then added. The aqueous layer was extracted with ethyl acetate. The organic layer was washed with saturated aqueous sodium chloride, and then dried over anhydrous sodium sulfate. After the organic layer was concentrated under reduced pressure, the resulting residue was purified by silica gel column chromatography (hexane/ethyl acetate) to afford 2-{[(3,6-dichloro-5-methylpyridazin-4-yl)methyl][(4-methoxyphenyl)methyl]amino}ethan-1-ol (1.326 g) as a solid.

### Production Example 72

A mixture of (1R,2R)-2-[(2-{[(3,6-dichloro-5-methylpyridazin-4-yl)methyl]sulfanyl}ethyl)amino]cyclohexan-1-ol (0.217 g), DMF (6 mL), potassium carbonate (0.171 g), and copper(I) iodide (0.236 g) was stirred at 150°C for 1 h under microwave irradiation. The reaction mixture was filtered through Celite, water was added to the filtrate, and the mixture was filtered through Celite again. The aqueous layer was extracted from the filtrate with ethyl acetate, and the organic layer was washed sequentially with water and saturated aqueous sodium chloride and then dried over anhydrous sodium sulfate. The organic layer was concentrated under reduced pressure and the resulting residue was purified by silica gel column chromatography (hexane/ethyl acetate) to afford (1R,2R)-2-(3-chloro-4-methyl-7,8-dihydropyridazino[3,4-e][1,4]thiaazepin-9(5H)-yl)cyclohexan-1 all (0.040 g) as a solid.

### Production Example 73

A mixture of 4-(bromomethyl)-3,6-dichloro-5-methylpyridazine (2.026 g), DMF (28 mL), cesium carbonate (2.711 g), and mercaptoethanol (0.59 mL) was stirred at 60 to 70°C for 3 hours. The reaction mixture was poured into water. The aqueous layer was then extracted with ethyl acetate. The organic layer was washed sequentially with water and saturated aqueous sodium chloride. The organic layer was dried over anhydrous sodium sulfate and the organic layer was concentrated under reduced pressure. The resulting residue was purified by silica gel column chromatography (hexane/ethyl acetate) to afford 2-{[(3,6-dichloro-5-methylpyridazin-4-yl)methyl]sulfanyl}ethan-1-ol (0.671 g) as an oil.

### Production Example 74

A mixture of 2-{[(3,6-dichloro-5-methylpyridazin-4-yl)methyl](methyl)amino}ethan-1-ol (0.623 g) and dichloromethane (2.5 mL) was admixed with triethylamine (0.42 mL) and p-toluenesulfonyl chloride (0.475 g) with stirring under ice-cold conditions. The mixture was stirred for 42 hours while the temperature was allowed to rise spontaneously to room temperature. The reaction mixture was poured into saturated aqueous ammonium chloride, and the aqueous layer was extracted with dichloromethane and the organic layer was dried over anhydrous sodium sulfate. After the organic layer was concentrated under reduced pressure, the resulting residue was purified by silica gel column chromatography (hexane/ethyl acetate) to afford 2-chloro-N-[(3,6-dichloro-5-methylpyridazin-4-yl)methyl]-N-(methyl)ethan-1-amine (0.212 g) as an oil.

### Production Example 75

A mixture of N-[3-(3,6-dichloro-5-methylpyridazin-4-yl)propyl]-1-(oxan-2-yl)-1H-pyrazol-3-amine (0.513 g), triethylamine (0.77 mL), and DMSO (3 mL) was stirred at 150°C for 6 hours under microwave irradiation. The reaction mixture was poured into water, and the aqueous layer was extracted with ethyl acetate. The organic layer was combined and washed with water and saturated sodium chloride solution, and dried over anhydrous sodium sulfate. After the organic layer was concentrated under reduced pressure, the resulting residue was purified by silica gel column chromatography (hexane/ethyl acetate). A mixture of the purified product (0.219 g), potassium carbonate (0.163 g), copper(I) iodide (0.225 g), and DMF (6 mL) was stirred at 150°C for 1 hour under microwave irradiation. The reaction mixture was poured into water. The aqueous layer was then extracted with ethyl acetate. The organic layer was washed sequentially with water and saturated aqueous sodium chloride, and then dried over anhydrous sodium sulfate. The organic layer was concentrated under reduced pressure and the resulting residue was purified by silica gel column chromatography (hexane/ethyl acetate) to afford 3-chloro-4-methyl-8-[1-(oxan-2-yl)-1H-pyrazol-3-yl]-5,6,7,8-tetrahydropyrido[2,3-c]pyridazine (0.025 g) as a solid.

### Production Example 76

A mixture of [(3R)-1-(2,2-difluoroethyl)piperidin-3-yl]tert-butyl carbamate (0.512 g), hydrogen chloride 1,4-dioxane solution (4 M, 5 mL), and methanol (10 mL) was stirred at room temperature for 4 hours. After hydrogen chloride 1,4-dioxane solution (4 M, 5 mL) was added, the mixture was stirred at room temperature for 42 hours. The reaction mixture was concentrated under reduced pressure and azeotroped with toluene to afford (3R)-1-(2,2-difluoroethyl)piperidin-3-amine dihydrochloride (0.461 g) as a solid.

### Production Example 77

A mixture of (3R)-piperidin-3-yl tert-butyl carbamate (2.163 g), 2,2-difluoroethyl trifluoromethanesulfonate (1.7 mL), DIPEA (3.7 mL), and 1,4-dioxane (36 mL) was stirred at 80°C for 20 hours. The reaction mixture was allowed to cool to room temperature, concentrated under reduced pressure, and purified by silica gel column chromatography (hexane/ethyl acetate) to afford [(3R)-1-(2,2-difluoroethyl)piperidin-3-yl]tert-butyl carbamate (2.779 g) as an oil.

### Production Example 78

A mixture of 4-bromo-3-(methoxymethoxy)benzaldehyde (1.086 g), N,N-diethylaminosulfur trifluoride (1.77 mL), and dichloromethane (15 mL) was stirred at 0°C for 30 min and then at room temperature for 4 hours. After the reaction mixture was cooled to 0°C, saturated sodium bicarbonate solution was added and the aqueous layer was extracted with dichloromethane. The organic layer was combined and then dried over anhydrous sodium sulfate, and the organic layer was concentrated under reduced pressure. The resulting residue was purified by silica gel column chromatography (hexane/ethyl acetate) to afford 1-bromo-4-(difluoromethyl)-2-(methoxymethoxy)benzene (0.973 g) as an oil.

### Production Example 79

A mixture of 2-{(7R)-9-[(1R,2R)-2-hydroxycyclohexyl]-6-[(4-methoxyphenyl)methyl]-4,7-dimethyl-6,7,8,9-tetrahydro-5H-pyridazino[3,4-e][1,4]diazepin-3-yl}-5-(trifluoromethyl)phenol (0.590 g), trifluoroacetic acid (4 mL), and anisole (1 mL) was stirred while heating under reflux for 24 hours. The reaction mixture was concentrated under reduced pressure, and the resulting residue was admixed with saturated aqueous sodium bicarbonate, ethyl acetate, and methanol, and the aqueous layer was then extracted with ethyl acetate. The organic layer was washed with saturated aqueous sodium chloride, and then dried over anhydrous sodium sulfate. After the organic layer was concentrated under reduced pressure, the resulting residue was purified by silica gel column chromatography (dichloromethane/methanol) to afford 2-{(7R)-9-[(1R,2R)-2-hydroxycyclohexyl]-4,7-dimethyl-6,7,8,9-tetrahydro-5H-pyridazino[3,4 -e][1,4]diazepin-3-yl}-5-(trifluoromethyl)phenol (0.256 g) as a solid.

### Production Example 80

Under nitrogen atmosphere, a mixture of (3S)-3-(3-chloro-4-methyl-5,6-dihydro-7H-pyrrolo[2,3-c]pyridazin-7-yl)pyrrolidin-2-one (320 mg), 1,2-dimethoxyethane (3 mL), and water (1 mL) was admixed with 2-[4-(difluoromethoxy)-2-(methoxymethoxy)phenyl]-4,4,5,5-tetramethyl-1,3,2-dioxaborolane (627 mg), sodium carbonate (403 mg), and SPhos Pd G3 (98.8 mg) at 25°C, and was then stirred at 120°C for 2 h under microwave irradiation. The reaction mixture was admixed with water (4 mL), and was then extracted with ethyl acetate. The resulting organic layer was washed with saturated aqueous sodium chloride, and then dried over anhydrous sodium sulfate. After the solution was concentrated under reduced pressure, the resulting residue was purified by silica gel column chromatography (dichloromethane/methanol) to afford (3S)-3-{3-[4-(difluoromethoxy)-2-(methoxymethoxy)phenyl]-4-methyl-5,6-dihydro-7H-pyrrolo[2,3-c] pyridazin-7-yl}pyrrolidin-2-one (103 mg) as a solid.

### Example 1

A mixture of (1R,2R)-2-(3-chloro-4-methyl-6,7-dihydropyrido[2,3-c]pyridazin-8(5H)-yl)cyclohexan-1-ol (113 mg), 1,4-dioxane (3.5 mL), and water (0.7 mL) was admixed with potassium carbonate (171 mg), RuPhos Pd G3 (50 mg), and [2-hydroxy-4-(trifluoromethyl)phenyl]boronic acid (251 mg), and was then stirred at 100°C for 1.5 hours under microwave irradiation. The reaction mixture was admixed with water and then extracted with chloroform. The organic layer was washed with saturated aqueous sodium chloride, and then dried over anhydrous magnesium sulfate. After the solution was concentrated under reduced pressure, the resulting residue was purified by silica gel column chromatography (hexane/ethyl acetate while using neutral silica gel, followed by chloroform/methanol while using basic silica gel). The purified product was admixed with diethyl ether (2 mL) and hexane (5 mL) and the solid was filtered off to afford 2-{8-[(1R,2R)-2-hydroxycyclohexyl]-4-methyl-5,6,7,8-tetrahydropyrido[2,3-c]pyridazin-3-yl}-5-(trifluoromethyl)phenol (46 mg) as a solid.

### Example 2

A mixture of [2-hydroxy-4-(trifluoromethyl)phenyl]boronic acid (35 mg), 3-chloro-4-methyl-8-[(3R)-1-methylpiperidin-3-yl]-5,6,7,8-tetrahydropyrido[2,3-c]pyridazine (24 mg), potassium carbonate (36 mg), 1,4-dioxane (2 mL), and water (0.5 mL) was admixed with RuPhos Pd G3 (7 mg) at room temperature, and was then stirred at 100°C for 1 hour. The reaction mixture was allowed to cool to room temperature, admixed with silica gel, and then concentrated under reduced pressure. The resulting residue was purified by silica gel column chromatography (chloroform/methanol while using neutral silica gel, followed by chloroform/methanol while using basic silica gel) to afford 2-{4-methyl-8-[(3R)-1-methylpiperidin-3-yl]-5,6,7,8-tetrahydropyrido[2,3-c]pyridazin-3-yl}-5-(trifluoromethyl)phenol (7.1 mg) as a solid.

### Example 3

Under nitrogen atmosphere, a mixture of 3-chloro-8-[(3R)-1-ethylpiperidin-3-yl]-4-methyl-5,6,7,8-tetrahydropyrido[2,3-c]pyridazine (200 mg), [2-hydroxy-4-(trifluoromethyl)phenyl]boronic acid (210 mg), 1,2-dimethoxyethane (8 mL), and water (1 mL) was admixed with sodium carbonate (216 mg) and SPhos Pd G3 (52.9 mg) at 25°C, and was then stirred at 100°C for 16 hours. The reaction mixture was concentrated under reduced pressure and the resulting residue was purified by reverse-phase preparative HPLC (ODS column; aqueous ammonia + aqueous ammonium bicarbonate/acetonitrile) to afford 2-{8-[(3R)-1-ethylpiperidin-3-yl]-4-methyl-5,6,7, 8-tetrahydropyrid[2,3-c]pyridazin-3-yl }-5-(trifluoromethyl)phenol (65.7 mg) as a solid.

### Example 4

Under argon atmosphere, a mixture of (1R,2R)-2-(3-chloro-4-methyl-7,8-dihydropyridazino[3,4-e][1,4]oxaazepin-9(5H)-yl)cyclohexane-1-ol (90 mg), [2-hydroxy-4-(trifluoromethyl)phenyl]boronic acid (120 mg), RuPhos Pd G3 (38 mg), potassium carbonate (105 mg), 1,4-dioxane (2 mL), and water (0.4 mL) was stirred at 100°C for 1 hour under microwave irradiation. The reaction mixture was concentrated under reduced pressure and the resulting residue was purified by silica gel column chromatography (chloroform/methanol while using basic silica gel, then chloroform/methanol while using neutral silica gel). The purified product was suspended in hexane/ethyl acetate (15/1) and the solid was filtered off to afford 2-{9-[(1R,2R)-2-hydroxycyclohexyl]-4-methyl-5,7,8,9-tetrahydropyridazino[3,4-e][1,4]oxaazepin-3-yl}-5-(trifluoromethyl)phenol (76 mg) as a solid.

### Example 5

Under argon atmosphere, a mixture of (3S)-3-(3-chloro-4-methyl-5,6-dihydro-7H-pyrrolo[2,3-c]pyridazin-7-yl)pyrrolidin-2-one (100 mg), 1,4-dioxane (3 mL), [2-hydroxy-4-(trifluoromethyl)phenyl]boronic acid (163 mg), RuPhos Pd G3 (33 mg), potassium carbonate (164 mg), and water (0.6 mL) was stirred at 100°C for 1 hour under microwave irradiation. The reaction mixture was cooled to room temperature, admixed with chloroform/methanol (9/1) and saturated aqueous sodium chloride/water (1/1), and then stirred at room temperature for 10 minutes. The organic layer was dried over anhydrous sodium sulfate. After the solution was concentrated under reduced pressure, the resulting residue was purified by silica gel column chromatography (chloroform/methanol while using neutral silica gel, followed by chloroform/methanol while using basic silica gel) to afford (3S)-3-{3-[2-hydroxy-4-(trifluoromethyl)phenyl]-4-methyl-5,6-dihydro-7H-pyrrolo[2,3-c]pyridazin-7-yl } pyrrolidin-2-one (88 mg) as a solid.

### Example 6

A mixture of 3-chloro-4-methyl-9-[(3R)-1-methylpiperidin-3-yl]-5,7,8,9-tetrahydropyridazino[3,4-e][1,4]oxaazepine (59 mg), 1,4-dioxane (0.8 mL), 5-methoxy-2-(4,4,5,5-tetramethyl-1,3,2-dioxaborolan-2-yl)phenol (150 mg), RuPhos Pd G3 (26 mg), potassium carbonate (83 mg), and water (0.2 mL) was stirred at 100°C for 1 hour under microwave irradiation. The reaction mixture was admixed with chloroform and saturated aqueous sodium chloride/water (1/1), and the organic layer was then dried over anhydrous sodium sulfate. After the solution was concentrated under reduced pressure, the resulting residue was purified by silica gel column chromatography (chloroform/methanol while using basic silica gel, followed by chloroform/methanol while using neutral silica gel) to afford 5-methoxy-2-{4-methyl-9-[(3R)-1 methylpiperidin-3-yl]-5,7,8,9-tetrahydropyridazino[3,4-e][1,4]oxaazepin-3-yl}phenol (50 mg) as a solid.

### Example 7

Under argon atmosphere, a mixture of (1R,2R)-2-(3-chloro-4,6-dimethyl-5,6,7,8-tetrahydro-9H-pyridazino[3,4-e][1,4]diazepin-9-yl)cyclohexane-1-ol (0.152 g), [2-hydroxy-4-(trifluoromethyl)phenyl]boronic acid (0.201 g), PdCl₂(dppf)·CH₂Cl₂ (0.040 g), potassium carbonate (0.135 g), 1,4-dioxane (5 mL), and water (1.2 mL) was stirred at 45°C for 1.5 hours, followed by 13.5 hours at 120°C. The reaction mixture was allowed to cool to room temperature, and was then poured into water. The aqueous layer was extracted with ethyl acetate. The organic layer was washed with saturated aqueous sodium chloride, and then dried over anhydrous sodium sulfate. After the organic layer was concentrated under reduced pressure, the resulting residue was purified by silica gel column chromatography (ethyl acetate/methanol) to afford 2-{9-[(1R,2R)-2-hydroxycyclohexyl]-4,6-dimethyl-6,7,8,9-tetrahydro-5H-pyridazino[3,4-e][1,4]diazepin-3-yl}-5-(trifluoromethyl)phenol (0.047 g) as a solid.

### Example 27

Under nitrogen atmosphere, a mixture of 2-[(3R)-3-(3-chloro-4-methyl-6,7-dihydropyrido[2,3-c]pyridazin-8(5H)-yl)piperidin-1-yl]ethan-1-ol (150 mg), 1,2-dimethoxyethane (1.5 mL), and water (1.5 mL) was admixed with [2-hydroxy-4-(trifluoromethyl)phenyl]boronic acid (149 mg), sodium carbonate (153 mg), and SPhos Pd G3 (37.7 mg) at 25°C, and was then stirred at 120°C for 2 h under microwave irradiation. The reaction mixture was admixed with water, and was extracted with ethyl acetate. The organic layer was washed with saturated aqueous sodium chloride, and then dried over anhydrous sodium sulfate. After the solution was concentrated under reduced pressure, the resulting residue was purified by reverse-phase preparative HPLC (ODS column; hydrochloric acid/acetonitrile) to afford 2-{8-[(3R)-1-(2-hydroxyethyl)piperidin-3-yl]-4-methyl-5, 6,7, 8-tetrahydropyrid[2,3-c]pyridazin-3-yl }-5-(trifluoromethyl)phenol dihydrochloride (77.6 mg) as a solid.

### Example 28

Under nitrogen atmosphere, a mixture of (1R,2R)-2-(3-chloro-4-methyl-6,7-dihydropyrido[2,3-c]pyridazin-8(5H)-yl)cyclohexan-1-ol (153 mg), 2-[4-(difluoromethoxy)-2-(methoxymethoxy)phenyl]-4,4,5,5-tetramethyl-1,3,2-dioxaborolane (359 mg), RuPhos Pd G3 (46 mg), 1,4-dioxane (4 mL), water (1 mL), and potassium carbonate (226 mg) was stirred at 105°C for 3 hours. The mixture was cooled to room temperature, admixed with chloroform and water, and then extracted with chloroform. The organic layer was concentrated under reduced pressure. The resulting residue was admixed with methanol (3 mL) and 12 M hydrochloric acid (0.5 mL) at room temperature, and the mixture was stirred at 60°C for 2 hours. The reaction mixture was admixed with sodium hydroxide aqueous solution (1 M, 6 mL) and water (4 mL) at room temperature, and then extracted with chloroform/2-propanol (5/1). The organic layer was concentrated under reduced pressure. The resulting residue was purified by silica gel column chromatography (chloroform/methanol). The resulting purified product was admixed with diisopropyl ether (2 mL) and hexane (10 mL) at room temperature and triturated. The solid was filtered off and dried under reduced pressure to afford 5-(difluoromethoxy)-2-{8-[(1R,2R)-2-hydroxycyclohexyl]-4-methyl-5,6,7,8-tetrahydropyrido[2,3-c]pyridazin-3-yl}phenol (140 mg) as a solid.

### Example 29

Under nitrogen atmosphere, a mixture of (3 S,4R)-4-(3-chloro-4-methyl-6,7-dihydropyrido[2,3-c]pyridazin-8(5H)-yl)oxan-3-ol (123 mg), 2-[4-(difluoromethoxy)-2-(methoxymethoxy)phenyl]-4,4,5,5-tetramethyl-1,3,2-dioxaborolane (286 mg), RuPhos Pd G3 (37.2 mg), 1,4-dioxane (4 mL), water (1 mL), and potassium carbonate (180 mg) was stirred at 105°C for 3 hours. The mixture was cooled to room temperature, admixed with chloroform and water, and then extracted with chloroform. The organic layer was concentrated under reduced pressure. The resulting residue was admixed with methanol (3 mL) and hydrochloric acid (12 M, 0.5 mL) at room temperature, and the mixture was stirred at 60°C for 2 hours. The reaction mixture was admixed with sodium hydroxide aqueous solution (1 M, 6 mL) and water (4 mL) at room temperature, and then extracted with chloroform/2-propanol (5/1). The organic layer was concentrated under reduced pressure. The resulting residue was purified by silica gel column chromatography (chloroform/methanol). The resulting purified product was admixed with diisopropyl ether (2 mL) and hexane (10 mL) at room temperature and triturated. The solid was filtered off and dried under reduced pressure to afford (3S,4R)-4-{3-[4-(difluoromethoxy)-2-hydroxyphenyl]-4-methyl-6,7-dihydropyrido[2,3-c]pyridazin-8(5H)-yl}oxan-3-ol (127 mg) as a solid.

### Example 30

A mixture of (1R,2R)-2-{3-[4-(difluoromethoxy)-2-(methoxymethoxy)phenyl]-4-methyl-7,8-dihydropyridazino[3,4-e][1,4]oxaazepin-9(5H)-yl}cyclohexan-1-ol (110 mg) and methanol (3 mL) was admixed with hydrochloric acid (12 M, 0.3 mL) at room temperature, and was then stirred at 60°C for 1.5 hours. The mixture was allowed to cool to room temperature, and was then concentrated under reduced pressure. The resulting residue was admixed with chloroform/methanol (10/1) and basic silica gel, and the mixture was concentrated under reduced pressure. The resulting residue was purified by silica gel column chromatography (basic silica gel, chloroform/methanol). The resulting purified product was suspended in hexane/ethyl acetate (20/1). The solid was filtered off and dried under reduced pressure to afford 5-(difluoromethoxy)-2- f 9-[(1R,2R)-2-hydroxycyclohexyl]-4-methyl-5,7,8,9-tetrahydropyridazino[3,4-e][1,4]oxaazepin-3-yl}phenol (72 mg) as a solid.

### Example 31

A mixture of (3R)-3-{3-[2-hydroxy-4-(trifluoromethyl)phenyl]-4-methyl-6,7-dihydropyrido[2,3-c]pyridazin-8(5H)-yl}piperidine-1-*tert*-butyl carboxylic acid (7.4 g) and dichloromethane (100 mL) was admixed with trifluoroacetic acid (103 mL) at 25°C, and was then stirred at the same temperature for 16 hours. The reaction mixture was concentrated under reduced pressure. A mixture of a portion of the residue obtained (386 mg) and methanol (3 mL) was admixed with triethylamine (0.106 mL) to bring the pH to about 8, and glacial acetic acid (45.9 mg) was then added to bring the pH to about 5. The resulting mixture was admixed with [(1-ethoxycyclopropyl)oxy]tri(methyl)silane (267 mg) and tetraisopropyl ortho-titanate (1.09 g), and was then stirred at 25°C for 30 minutes. The resulting mixture was admixed with sodium cyanoborohydride (240 mg), and was then stirred at 60°C for 16 hours. The reaction mixture was admixed with saturated sodium bicarbonate aqueous solution (3 mL), and was then extracted with dichloromethane. The organic layer was dried over anhydrous sodium sulfate. After the solution was concentrated under reduced pressure, the resulting residue was purified by reverse-phase preparative HPLC (ODS column, ammonia water/acetonitrile) to afford 2-{8-[(3R)-1-cyclopropylpiperidin-3-yl]-4-methyl-5,6,7,8-tetrahydropyrido[2,3-c]pyridazin-3-yl 1-5-(trifluoromethyl)phenol (97.9 mg) as a solid.

### Example 32

A mixture of (3R)-3-{3-[2-hydroxy-4-(trifluoromethyl)phenyl]-4-methyl-6,7-dihydropyrido[2,3-c]pyridazin-8(5H)-yl}piperidine-1-*tert*-butyl carboxylic acid (7.4 g) and dichloromethane (100 mL) was admixed with trifluoroacetic acid (103 mL) at 25°C, and was then stirred at the same temperature for 16 hours. The reaction mixture was concentrated under reduced pressure. A portion of the residue obtained (400 mg) was admixed with triethylamine (0.220 mL) and dichloromethane (4 mL), followed by methyl chloroformate (0.082 mL) at 25°C, and the mixture was stirred at the same temperature for 16 hours. The reaction mixture was admixed with water, and was then extracted with ethyl acetate. The organic layer was washed with saturated aqueous sodium chloride, and then dried over anhydrous sodium sulfate. After the solution was concentrated under reduced pressure, the resulting residue was purified by reverse-phase preparative HPLC (ODS column, ammonia water/acetonitrile) to afford (3R)-3-{3-[2-hydroxy-4-(trifluoromethyl)phenyl]-4-methyl-6,7-dihydropyrido[2,3-c]pyridazin-8(5H)-yl } piperidine-1-methyl carboxylate (138 mg) as a solid.

### Example 33

Under nitrogen atmosphere, a mixture of 2-{4-methyl-8-[(3R)-piperidin-3-yl]-5,6,7,8-tetrahydropyrido[2,3-c]pyridazin-3-yl}-5-(trifluoromethyl)phenol (250 mg) and dichloromethane (10 mL) was admixed with triethylamine (0.133 mL) and isocyanato tri(methyl)silane (0.093 mL) at 20°C, and was then stirred at the same temperature for 16 hours. The reaction mixture was admixed with water and then extracted with dichloromethane. The organic layer was concentrated under reduced pressure. The residue obtained and 2-{ 4-methyl-8-[(3R)-piperidin-3-yl]-5,6, 7,8-tetrahydropyrido[2,3-c]pyridazin-3-yl}-5-(trifluoromethyl)phenol (50 mg) were used in the same manner as above and the resulting residue was combined and then purified by silica gel column chromatography (dichloromethane/methanol) to afford (3R)-3-{3-[2-hydroxy-4-(trifluoromethyl)phenyl]-4-methyl-6,7-dihydropyrido[2,3-c]pyridazin-8(5H)-yl}piperidin-1-carboxyamide (144 mg) as a solid.

### Example 34

A mixture of 2-{4-methyl-8-[(3R)-piperidin-3-yl]-5,6,7,8-tetrahydropyrido[2,3-c]pyridazin-3-yl}-5-(trifluoromethyl)phenol (50 mg) and dichloromethane (2 mL) was admixed with DIPEA (0.067 mL) and methanesulfonic anhydride (33.3 mg) at 0°C, and was then stirred at 25°C for 16 hours. The reaction mixture was concentrated under reduced pressure. The resulting residue and the residue obtained by performing the above procedure two more times were each combined and purified by reverse-phase preparative HPLC (ODS column, aqueous ammonia + aqueous ammonium bicarbonate/acetonitrile) to afford 2-{8-[(3R)-1-(methansulfonyl)piperidin-3-yl]-4-methyl-5,6,7,8-tetrahydropyrido[2,3-c]pyridazin-3-yl}-5-(trifluoromethyl)phenol (107 mg) as a solid.

### Example 35

A mixture of (1s,3s)-3-amino-1-methylcyclobutan-1-ol monohydrochloride (118 mg) and NMP (1.5 mL) was admixed with triethylamine (0.46 mL) at room temperature, and was then stirred at the same temperature for 5 minutes. The resulting mixture was admixed with 2-(3,6-dichloro-5-methylpyridazin-4-yl)ethyl 4-methylbenzene-1-sulfonate (200 mg) at room temperature, and was then stirred at 150°C for 1 hour under microwave irradiation. The mixture was cooled to room temperature, and then admixed with water and ethyl acetate. The mixture was extracted with ethyl acetate. The organic layer was dried over anhydrous magnesium sulfate and the solution was concentrated under reduced pressure. The resulting residue was admixed with 1,4-dioxane (8 mL), water (2 mL), [2-hydroxy-4-(trifluoromethyl)phenyl]boronic acid (230 mg), RuPhos Pd G3 (47.1 mg), and potassium carbonate (241 mg), and the mixture was stirred at 105°C for 2 hours. The mixture was cooled to room temperature, admixed with water, and then extracted with chloroform/2-propanol (5/1). The organic layer was concentrated under reduced pressure. The resulting residue was purified by silica gel column chromatography (chloroform/methanol). The purified product obtained was admixed with 1,4-dioxane (8 mL), water (2 mL), [2-hydroxy-4-(trifluoromethyl)phenyl]boronic acid (229 mg), RuPhos Pd G3 (47 mg), and potassium carbonate (240 mg), and the mixture was then stirred at 105°C for 2 hours under microwave irradiation. The mixture was cooled to room temperature, admixed with water, and then extracted with chloroform/2-propanol (5/1). The organic layer was concentrated under reduced pressure. The resulting residue was purified by silica gel column chromatography (chloroform/methanol). The resulting purified product was admixed with diisopropyl ether (2 mL) and hexane (10 mL) at room temperature and triturated. The solid was filtered off and dried under reduced pressure to afford 2-{7-[(1s,3s)-3-hydroxy-3-methylcyclobutyl]-4-methyl-6,7-dihydro-5H-pyrrolo[2,3-c]pyridazin-3-yl}-5-(trifluoromethyl)phenol (84.8 mg) as a solid.

### Example 36

A mixture of 2-[5-(aminomethyl)-6-{[(1R,2R)-2-hydroxycyclohexyl]amino}-4-methylpyridazin-3-yl]-5-(trifluoromethyl)phenol (0.054 g), formaldehyde (37% aqueous solution, 61 µL), and methanol (1.4 mL) was stirred at room temperature for 40 minutes, admixed with sodium triacetoxyborohydride (0.086 g), and then stirred at room temperature for 4 hours. Saturated sodium bicarbonate solution was added to the reaction mixture, and the aqueous layer was then extracted with ethyl acetate. The organic layer was combined and dried over anhydrous sodium sulfate. After the organic layer was concentrated under reduced pressure, the resulting residue was purified by silica gel column chromatography (dichloromethane/methanol) to afford 2- f 8-[(1R,2R)-2-hydroxycyclohexyl]-4,6-dimethyl-5,6,7,8-tetrahydropyrimido[4,5-c]pyridazin-3-yl}-5-(trifluoromethyl)phenol (0.028 g) as a solid.

### Example 37

A mixture of 2-[5-(aminomethyl)-6-{[(1R,2R)-2-hydroxycyclohexyl]amino}-4-methylpyridazin-3-yl]-5-(trifluoromethyl)phenol (0.037 g), carbonyldiimidazole (0.019 g), and THF (2 mL) was stirred at room temperature for 1 hour. The reaction mixture was admixed with ethyl acetate (6 mL) and hydrochloric acid (2 M, 6 mL), the aqueous layer was removed, and the organic layer was washed with hydrochloric acid (2 M). All the aqueous layers were combined, saturated sodium bicarbonate aqueous solution was added to make the solution basic, and the mixture was extracted with ethyl acetate. All the organic layers were combined and washed with saturated aqueous sodium chloride, and then dried over anhydrous sodium sulfate. After the organic layer was concentrated under reduced pressure, the resulting residue was purified by silica gel column chromatography (dichloromethane/methanol) to afford 8-[(1R,2R)-2-hydroxycyclohexyl]-3-[2-hydroxy-4-(trifluoromethyl)phenyl]-4-methyl-5,8-dihydropyrimido[4,5-c]pyridazin-7(6H)-one (0.010 g) as a solid.

### Example 38

A mixture of 2-{ 9-[(1R,2R)-2-hydroxycyclohexyl]-6-[(4-methoxyphenyl)methyl]-4-methyl-6,7,8,9-tetrahydro-5H-pyridazino[3,4-e][1,4]diazepin-3-yl}-5-(trifluoromethyl)phenol (0.053 g), trifluoroacetic acid (0.39 mL), and anisole (98 µL) was stirred at 80 to 100°C for 21 hours. After the reaction mixture was cooled to 0°C, saturated sodium bicarbonate solution was added to make the solution basic, and the aqueous layer was extracted with ethyl acetate. The organic layer was washed with saturated aqueous sodium chloride, and then dried over anhydrous sodium sulfate. After the organic layer was concentrated under reduced pressure, the resulting residue was purified by silica gel column chromatography (dichloromethane/methanol) to afford 2-{9-[(1R,2R)-2-hydroxycyclohexyl]-4-methyl-6,7,8,9-tetrahydro-5H-pyridazino[3,4-e][1,4]diazepin-3-yl}-5-(trifluoromethyl)phenol (0.010 g) as a solid.

### Example 39

A mixture of 2-{9-[(1R,2R)-2-hydroxycyclohexyl]-4-methyl-6,7,8,9-tetrahydro-5H-pyridazino[3,4-e][1,4]diazepin-3-yl}-5-(trifluoromethyl)phenol (0.123 g), DIPEA (61 µL), and dichloromethane (3 mL) was admixed with acetic anhydride (33 µL) with stirring under ice-cold conditions, and was then stirred at room temperature for 3.5 hours. The reaction mixture was admixed with water and the aqueous layer was then extracted with dichloromethane. The organic layer was dried over anhydrous sodium sulfate, the organic layer was concentrated under reduced pressure, and the resulting residue was purified by silica gel column chromatography (ethyl acetate/methanol) to afford (1R,2R)-2-{6-acetyl-3-[2-hydroxy-4-(trifluoromethyl)phenyl]-4-methyl-5, 6,7, 8-tetrahydro-9H-pyridazino[3,4-e][1,4]diazepin-9-yl} cyclohexyl acetate (0.082 g) as a solid.

### Example 40

A mixture of (1R,2R)-2-{6-acetyl-3-[2-hydroxy-4-(trifluoromethyl)phenyl]-4-methyl-5,6,7,8-tetrahydro-9H-pyridazino[3,4-e][1,4]diazepin-9-yl}cyclohexyl acetate (0.065 g), potassium carbonate (0.035 g), methanol (0.4 mL), and water (46 µL) was stirred at room temperature for 3.5 hours, and then stirred at 40 to 50°C for 2.5 hours. The reaction mixture was concentrated under reduced pressure, the residue was admixed with dichloromethane and 10% hydrochloric acid for dissolution, and the organic layer was then separated using a phase separator. The organic layer was dried over anhydrous sodium sulfate and the organic layer was concentrated under reduced pressure. The resulting residue was dissolved in dichloromethane, hexane was added, and the precipitated solid was filtered off to afford 1-{9-[(1R,2R)-2-hydroxycyclohexyl]-3-[2-hydroxy-4-(trifluoromethyl)phenyl]-4-methyl-5,7,8,9-tetrahydro-6H-pyridazino [3,4-e][1,4]diazepin-6-yl}ethan-1-one (0.056 g) as a solid.

### Example 41

A mixture of 2-{9-[(1R,2R)-2-hydroxycyclohexyl]-4-methyl-6,7,8,9-tetrahydro-5H-pyridazino[3,4-e][1,4]diazepin-3-yl}-5-(trifluoromethyl)phenol (0.091 g), acetaldehyde (18 µL), sodium triacetoxyborohydride (0.092 g), and methanol (2 mL) was stirred at room temperature for 3.5 hours. Saturated sodium bicarbonate aqueous solution was added to the reaction mixture, and the precipitated solid was filtered off. The resulting solid was purified by silica gel column chromatography (ethyl acetate/methanol) to afford 2-{6-ethyl-9-[(1R,2R)-2-hydroxycyclohexyl]-4-methyl-6,7,8,9-tetrahydro-5H-pyridazino[3,4-e][1,4]diazepin-3-yl}-5-(trifluoromethyl)phenol (0.012 g) as a solid.

### Example 43

A mixture of 2-{4-methyl-8-[1-(oxan-2-yl)-1H-pyrazol-3-yl]-5,6,7,8-tetrahydropyrido[2,3-c]pyridazin-3-yl}-5-(trifluoromethyl)phenol (0.039 g) and dichloromethane (1 mL) was admixed with trifluoroacetic acid (85 µL) with stirring under ice cold conditions, and was then stirred for 41 hours while the temperature was allowed to rise spontaneously to room temperature. The reaction mixture was cooled on ice, admixed with dichloromethane (1 mL) and trifluoroacetic acid (85 µL), and was then stirred for 6 hours while the temperature was allowed to rise spontaneously to room temperature. Next, the reaction mixture was cooled on ice, admixed with dichloromethane (1 mL) and trifluoroacetic acid (85 µL), and was then stirred for 66 hours while the temperature was allowed to rise spontaneously to room temperature. The reaction mixture was cooled to 0°C, saturated sodium bicarbonate solution was added, and the aqueous layer was then extracted with ethyl acetate. The organic layer was combined and washed with saturated aqueous sodium chloride, and then dried over anhydrous sodium sulfate. The organic layer was concentrated under reduced pressure. The solid obtained was purified by silica gel column chromatography (dichloromethane/methanol) to afford 2-[4-methyl-8-(1H-pyrazol-3-yl)-5,6,7,8-tetrahydropyrido[2,3-c]pyridazin-3-yl]-5-(trifluoromethyl)phenol (0. 016 g) as a solid.

### Example 44

A mixture of (1R,2R)-2-{3-[4-(difluoromethyl)-2-(methoxymethoxy)phenyl]-4,6-dimethyl-5,6,7,8-tetrahydro-9H-pyridazino[3,4-e][1,4]diazepin-9-yl}cyclohexane-1-ol (0.199 g), trifluoroacetic acid (0.72 mL), and dichloromethane (4.3 mL) was stirred at room temperature for 16.5 hours. After the reaction mixture was cooled to 0°C, saturated sodium bicarbonate solution was added and the aqueous layer was extracted with dichloromethane/methanol. The organic layer was dried over anhydrous sodium sulfate and the organic layer was concentrated under reduced pressure. The resulting residue was purified by silica gel column chromatography (chloroform/methanol). Dichloromethane and hexane were added to the resulting purified product, and the precipitated solid was filtered off to afford 5-(difluoromethyl)-2- f 9-[(1R,2R)-2-hydroxycyclohexyl]-4,6-dimethyl-6,7,8,9-tetrahydro-5H-pyridazino[3,4-e][1,4]diazepin-3-yl}phenol (0.067 g) as a solid.

### Example 45

A mixture of (3 S)-3-{3-[4-(difluoromethoxy)-2-(methoxymethoxy)phenyl]-4-methyl-5,6-dihydro-7H-pyrrolo[2,3-c]pyridazin-7-yl}pyrrolidin-2-one (80 mg) and 1,4-dioxane (2 mL) was admixed with 4 M hydrogen chloride 1,4-dioxane solution (2 mL) at 13°C, and was then stirred at 13°C to 20°C for 2 hours. The reaction mixture was concentrated under reduced pressure and the resulting residue was purified by reverse-phase preparative HPLC (ODS column, hydrochloric acid/acetonitrile) to afford (3S)-3-{3-[4-(difluoromethoxy)-2-hydroxyphenyl]-4-methyl-5,6-dihydro-7H-pyrrolo[2,3-c]pyridazin-7-yl}pyrrolidin-2-one (18 mg) as a solid.

Compounds of the Production Examples and Examples shown in the tables below were produced in substantially the same manner as in the above Production Examples or Examples.

**[Table 2-1]**

| PEx | Str | PEx | Str |
|---|---|---|---|
| 1 | 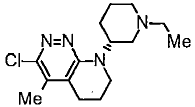 | 11 | 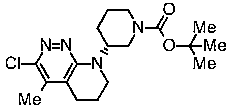 |
| 2 | 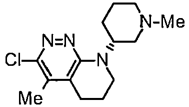 | 12 | 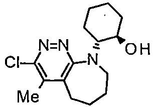 |
| 3 | 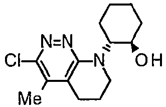 | 13 | 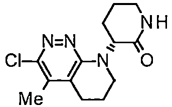 |
| 4 | 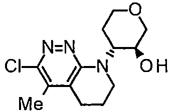 | 14 | 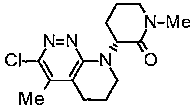 |
| 5 | 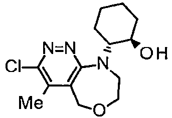 | 15 | 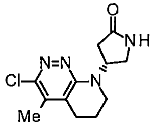 |
| 6 | 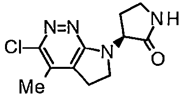 | 16 | 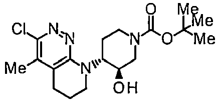 |
| 7 | 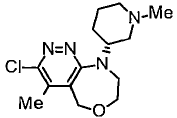 | 17 | 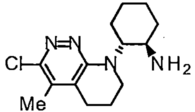 |
| 8 | 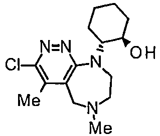 | 18 | 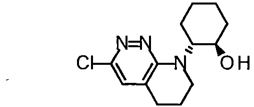 |
| 9 | 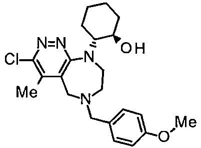 | 19 | 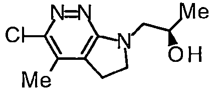 |
| 10 | 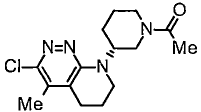 | 20 | 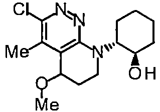 |

**[Table 2-2]**

| PEx | Str | PEx | Str |
|---|---|---|---|
| 21 | 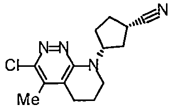 | 31 | 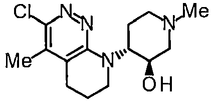 |
| 22 | 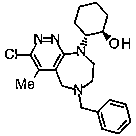 | 32 | 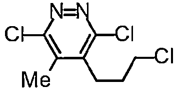 |
| 23 | 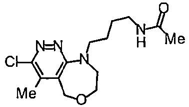 | 33 | 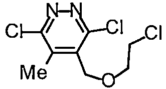 |
| 24 | 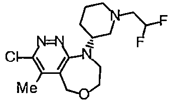 | 34 | 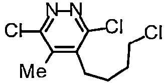 |
| 25 | 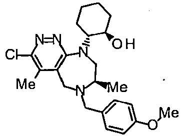 | 35 | 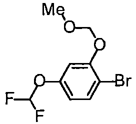 |
| 26 | 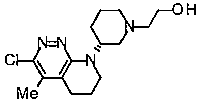 | 36 | 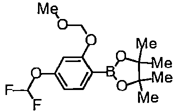 |
| 27 | 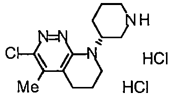 | 37 | 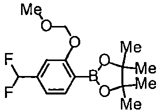 |
| 28 | 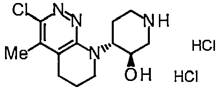 | 38 | 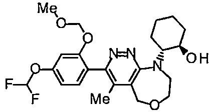 |
| 29 | 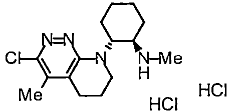 | 39 | 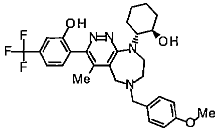 |
| 30 | 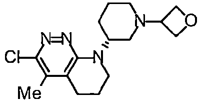 | 40 | 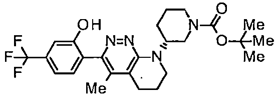 |

**[Table 2-3]**

| PEx | Str | PEx | Str |
|---|---|---|---|
| 41 | 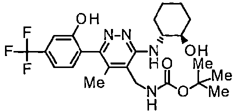 | 51 | 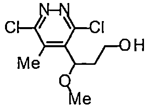 |
| 42 | 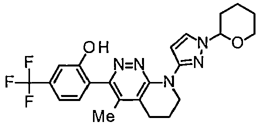 | 52 | 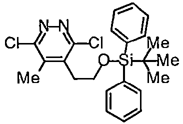 |
| 43 | 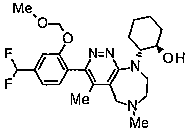 | 53 | 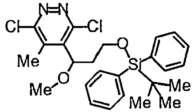 |
| 44 | 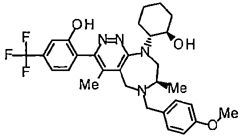 | 54 | 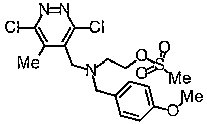 |
| 45 | 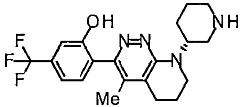 | 55 | 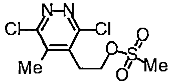 |
| 46 | 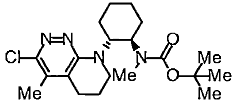 | 56 | 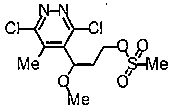 |
| 47 | 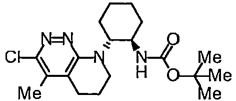 | 57 | 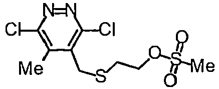 |
| 48 | 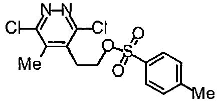 | 58 | 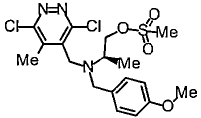 |
| 49 | 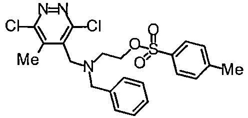 | 59 | 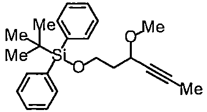 |
| 50 | 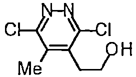 | 60 | 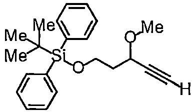 |

**[Table 2-4]**

| PEx | Str | PEx | Str |
|---|---|---|---|
| 61 | 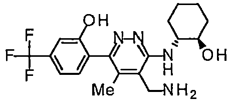 | 71 | 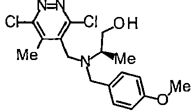 |
| 62 | 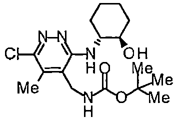 | 72 | 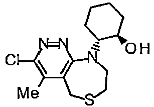 |
| 63 | 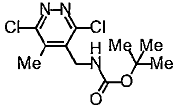 | 73 | 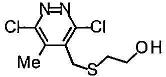 |
| 64 | 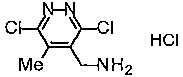 | 74 | 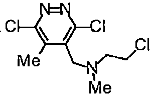 |
| 65 | 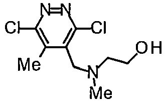 | 75 | 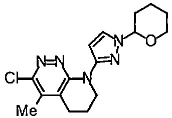 |
| 66 | 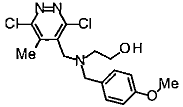 | 76 | 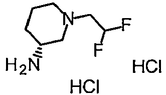 |
| 67 | 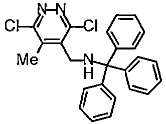 | 77 | 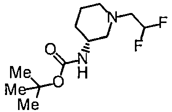 |
| 68 | 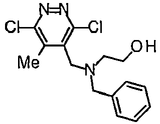 | 78 | 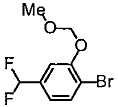 |
| 69 | 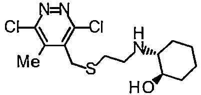 | 79 | 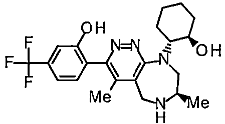 |
| 70 | 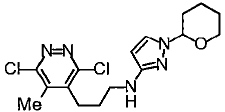 | 80 | 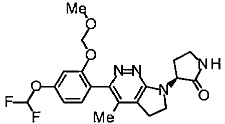 |

**[Table 3-1]**

| Ex | Str | Ex | Str |
|---|---|---|---|
| 1 | 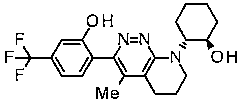 | 11 | 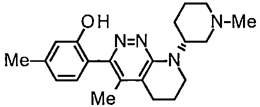 |
| 2 | 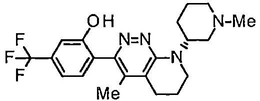 | 12 | 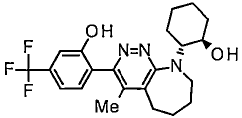 |
| 3 | 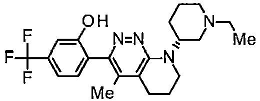 | 13 | 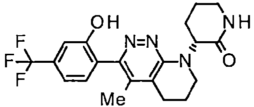 |
| 4 | 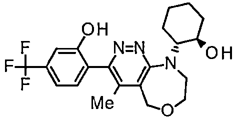 | 14 | 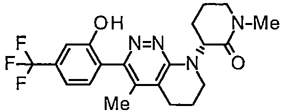 |
| 5 | 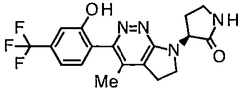 | 15 | 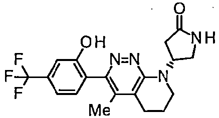 |
| 6 | 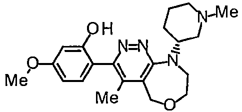 | 16 | 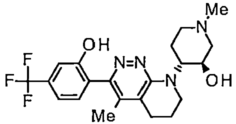 |
| 7 | 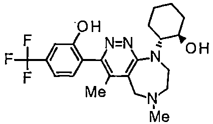 | 17 | 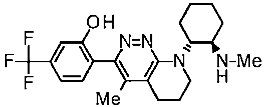 |
| 8 | 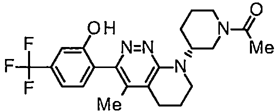 | 18 | 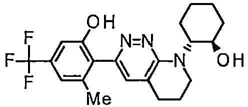 |
| 9 | 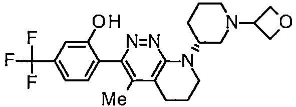 | 19 | 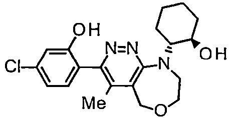 |
| 10 | 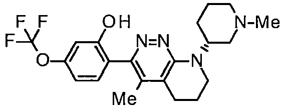 | 20 | 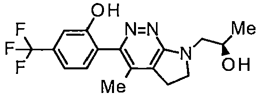 |

**[Table 3-2]**

| Ex | Str | Ex | Str |
|---|---|---|---|
| 21 | 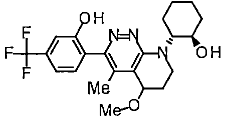 | 31 | 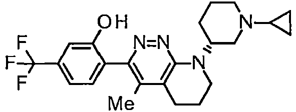 |
| 22 | 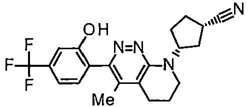 | 32 | 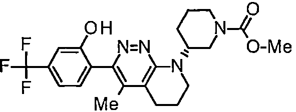 |
| 23 | 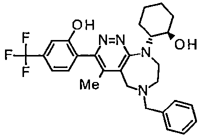 | 33 | 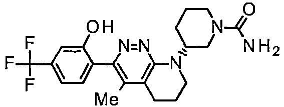 |
| 24 | 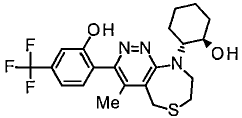 | 34 | 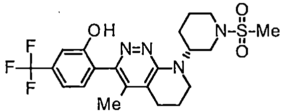 |
| 25 | 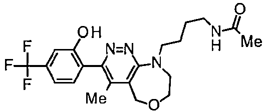 | 35 | 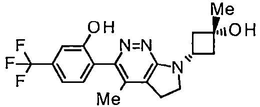 |
| 26 | 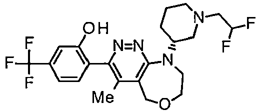 | 36 | 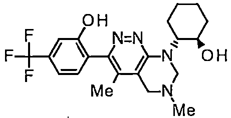 |
| 27 | 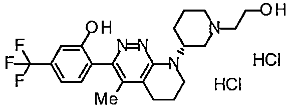 | 37 | 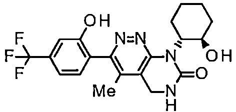 |
| 28 | 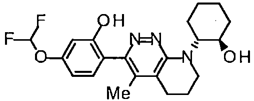 | 38 | 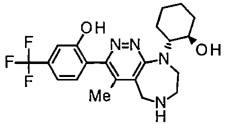 |
| 29 | 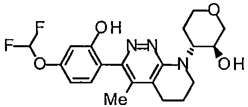 | 39 | 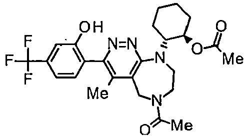 |
| 30 | 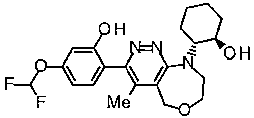 | 40 | 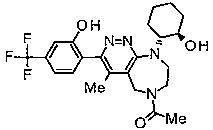 |

**[Table 3-3]**

| Ex | Str | Ex | Str |
|---|---|---|---|
| 41 | 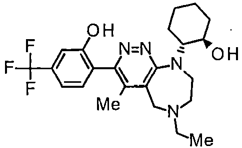 | 43 | 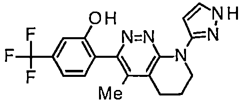 |
| 42 | 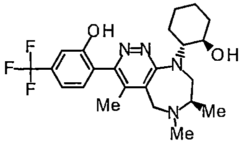 | 44 | 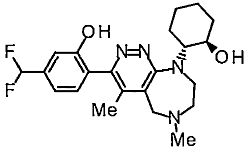 |
| | | 45 | 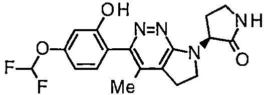 |

**[Table 4-1]**

| PEx | PSyn | DAT |
|---|---|---|
| 1 | - | ESI+; 295.1 |
| 2 | - | ESI+; 281.2, 283.2 |
| 3 | - | ESI+; 282.4, 284.4 |
| 4 | - | ESI+; 284.3, 286.4 |
| 5 | - | ESI+; 298.4, 300.4 |
| 6 | - | ESI+; 253.1, 255.1 |
| 7 | - | ESI+; 297.4, 299.3 |
| 8 | - | ESI+; 311.3, 313.2 |
| 9 | - | ESI+; 417.4, 419.3 |
| 10 | 1 | ESI+; 308.9 |
| 11 | 1 | ESI+; 367.1 |
| 12 | 1 | ESI+; 296.0 |
| 13 | 1 | ESI+; 281.2, 283.2 |
| 14 | 1 | ESI+; 295.2, 297.2 |
| 15 | 1 | ESI+; 267.1, 269.1 |
| 16 | 1 | ESI+; 383.2, 385.1 |
| 17 | 1 | ESI+; 281.1 |
| 18 | 1 | ESI+; 268.1 |
| 19 | 1 | ESI+; 228.1 |
| 20 | 1 | ESI+; 312.2, 314.0 |
| 21 | 1 | ESI+; 277.1, 279.1 |
| 22 | 1 | ESI+; 387.3, 389.3 |
| 23 | 1 | ESI+; 335.3, 337.2 [M+Na]+ |
| 24 | 1 | ESI+; 347.2, 349.2 |
| 25 | 1 | ESI+; 431.5, 433.5 |
| 26 | - | ESI+; 311.1 |
| 27 | - | ESI+; 267.1 |
| 28 | 27 | ESI+; 283.1, 285.1 |
| 29 | 27 | ESI+; 295.1 |
| 30 | - | ESI+; 323.1 |
| 31 | 30 | ESI+; 297.1 |
| 32 | - | ESI+; 239.0 |
| 33 | - | ESI+; 255.1 |
| 34 | 32 | ESI+; 254.9 |

**[Table 4-2]**

| PEx | PSyn | DAT |
|---|---|---|
| 35 | - | CI+; 282. 9 |
| 36 | - | ESI+; 353.2 [M+Na]+ |
| 37 | 36 | ¹H NMR (400 MHz, CDCl₃) δ ppm 1.36 (12 H, s) 3.52 (3 H, s) 5.22 (2 H, s) 6.61 (1 H, t, *J*=56.5 Hz) 7.13 - 7. 16 (2 H, m) 7.75 (1 H, d, *J*=7.3 Hz) |
| 38 | - | ESI+; 466.5 |
| 39 | - | ESI+; 543.4 |
| 40 | 38 | ESI+; 493.2 |
| 41 | 38 | ¹H NMR (400 MHz, DMSO-d₆) δ ppm 1.15 - 1.37 (4 H, m) 1.42 (9 H, s) 1.58 - 1.72 (3 H, m) 1.91 - 2.04 (2 H, m) 2.08 (3 H, s) 3.43 - 3.50 (1 H, m) 3.92 - 3.99 (1 H, m) 4.09 - 4.32 (2 H, m) 6.54 (1 H, br s) 7.21 (1 H, s) 7.24 (1 H, d, *J*=8.3 Hz) 7.37 (1 H, d, *J*=7.8 Hz) 7.62 - 7.65 (1 H, br m) 10.40 (1 H, br s) |
| 42 | 38 | ESI+; 460. 3 |
| 43 | 38 | ESI+; 463.5 |
| 44 | 38 | ESI+; 557.6 |
| 45 | - | ESI+; 393.4 |
| 46 | - | ESI+; 395. 2 |
| 47 | - | ESI+; 381. 2 |
| 48 | - | ESI+; 383.1, 385.1, 387.1 [M+Na]+ |
| 49 | 48 | ESI+; 502.2, 504.3 [M+Na]+ |
| 50 | - | ESI+; 207.1, 209.1 |
| 51 | 50 | ESI+; 250.9 |
| 52 | - | ESI+; 467.3, 469.3 [M+Na]+ |
| 53 | 52 | ESI+; 489.1 |
| 54 | - | ESI+; 456.1, 458.3 [M+Na]+ |
| 55 | - | ESI+; 307.2, 309.2, 311.1 [M+Na]+ |
| 56 | - | ESI+; 330.7 |
| 57 | - | ¹H NMR (400 MHz, CDCl₃) δ ppm 2.53 (3 H, s) 2.97 (2 H, t, *J*=6.4 Hz) 3.06 (3 H, s) 3.99 (2 H, s) 4.41 (2 H, t, *J*=6.9 Hz) |
| 58 | - | ESI+; 470.1, 472.3 [M+Na]+ |
| 59 | - | ESI+; 367. 2 |

**[Table 4-3]**

| PEx | PSyn | DAT |
|---|---|---|
| 60 | - | ¹H NMR (400 MHz, CDCl₃) δ ppm 1.07 (9 H, s), 1.97 - 2.01 (2 H, m), 2.43 (1 H, d, *J*=2.0 Hz), 3.42 (3 H, s), 3.73 - 3.91 (2 H, m), 4.25 - 4.30 (1 H, m), 7.39 - 7.44 (6 H, m), 7.67 - 7.70 (4 H, m) |
| 61 | - | ¹H NMR (400 MHz, CDCl₃) δ ppm 1.24 - 1.49 (4 H, m) 1.73 - 1.79 (2 H, m) 2.09 - 2.15 (2 H, m) 2.31 (3 H, s) 3.48 - 3.54 (1 H, m) 3.98 - 4.13 (3 H, m) 7.14 (1 H, d, *J*=7.4 Hz) 7.28 - 7.30 (2 H, m) |
| 62 | - | ¹H NMR (400 MHz, CDCl₃) δ ppm 1.34 - 1.44 (4 H, m) 1.46 (9 H, s) 1.69 - 1.76 (1 H, m) 2.04 - 2.11 (2 H, m) 2.32 - 2.33 (3 H, m) 3.44 - 3.50 (1 H, m) 3.97 - 4.02 (1 H, m) 4.27 - 4.29 (2 H, m) 4.87 - 4.93 (1 H, m) 6.33 (1 H, br s) |
| 63 | - | ¹H NMR (400 MHz, CDCl₃) δ ppm 1.42 (9 H, s) 2.63 (3 H, s) 4.48 (2 H, d, *J*=6.4 Hz) 5.14 (1 H, br s) |
| 64 | - | ¹H NMR (400 MHz, DMSO-d₆) δ ppm 2.57 (3 H, s) 4.23 (2 H, s) 8.68 (3 H, br s) |
| 65 | - | ESI+; 250.0, 252.0 |
| 66 | - | ESI+; 378.2, 380.1 [M+Na]+ |
| 67 | 65 | ¹H NMR (400 MHz, CDCl₃) δ ppm 1.90 (1 H, br s) 2.21 (3 H, s) 3.49 (2 H, s) 7.22 - 7.26 (3 H, m) 7.31 - 7.34 (6 H, m) 7.54 - 7.57 (6 H, m) |

**[Table 4-4]**

| PEx | PSyn | DAT |
|---|---|---|
| 68 | 65 | ESI+; 348.3, 350.3 [M+Na]+ |
| 69 | 65 | ¹H NMR (400 MHz, CDCl₃) δ ppm 0.93 - 1.02 (1 H, m) 1.18 - 1,33 (3 H, m) 1.71 - 1.74 (2 H, m) 2.01 - 2.07 (2 H, m) 2.17 - 2.23 (1 H, m) 2.52 (3 H, s) 2.75 - 2.80 (3 H, m) 2.99 - 3.07 (1 H, m) 3.14 - 3.20 (1 H, m) 3.90 (2 H, s) |
| 70 | 65 | ESI+; 392.1, 394.1 [M+Na]+ |
| 71 | 65 | ESI+; 392.1, 394.2 [M+Na]+ |
| 72 | - | ESI+; 314. 2 |
| 73 | - | ¹H NMR (400 MHz, CDCl₃) δ ppm 2.01 (1 H, br s) 2.53 (3 H, s) 2.83 (2 H, t, *J*=6.0 Hz) 3.86 (2 H, t, *J*=6.0 Hz) 3.95 (2 H, s) |
| 74 | - | ESI+; 268.0, 270.1 |
| 75 | - | ESI+; 334.2, 336.1 |
| 76 | - | ¹H NMR (400 MHz, CD₃0D) δ ppm 1.67 - 1.77 (1 H, m) 1.96 - 2.22 (3 H, m) 3.21 - 3.32 (2 H, m) 3.62 - 3.85 (5 H, m) 6.51 (1 H, tt, *J*=3.6, 53.2 Hz) |
| 77 | - | ¹H NMR (400 MHz, CDCl₃) δ ppm 1.45 (9 H, s) 1.53 - 1.71 (4 H, m) 2.43 - 2.54 (3 H, m) 2.66 - 2.74 (3 H, m) 3.74 (1 H, br s) 4.94 (1 H, br s) 5.85 (1 H, tt, *J*=4.0, 56.0 Hz) |
| 78 | - | ¹H NMR (400 MHz, CDCl₃) δ ppm 3.53 (3 H, s) 5.29 (2 H, s) 6.59 (1 H, t, *J*=56.5 Hz) 7.04 (1 H, d, *J*=7. 8 Hz) 7.29 (1 H, s) 7.63 (1 H, d, *J*=7.8 Hz) |
| 79 | - | ESI+; 437.2 |
| 80 | - | ESI+; 421. 1 |

**[Table 5-1]**

| Ex | Syn | DAT |
|---|---|---|
| 1 | - | ESI+; 408.2 |
| | | ¹H NMR (500 MHz, DMSO-*d*₆) δ ppm 1. 18 - 1. 37 (3 H, m), 1.40 - 1.58 (1 H, m), 1.62 - 1.81 (4 H, m), 1.91 (3 H, s), 1.93 - 2.05 (2 H, m), 2.56 - 2.66 (1 H, m), 2.66 - 2.75 (1 H, m), 3.26 - 3.44 (2 H, m), 3.63 (1 H, br s), 4.49 (1 H, d, *J*=5.0 Hz), 4. 76 (1 H, br s), 7.18 - 7. 20 (1 H, m), 7.20 - 7.24 (1 H, m), 7.37 (1 H, d, *J*=7.5 Hz), 10.35 (1 H, br s) |
| 2 | - | ESI+; 407. 2 |
| | | ¹H NMR (500 MHz, DMSO-d₆) δ ppm 1. 50 - 1. 65 (2 H, m), 1. 65 - 1.76 (2 H, m), 1.76 - 1. 89 (3 H, m), 1. 91 (3 H, s), 1.99 (1 H, t, *J*=10. 5 Hz), 2.19 (3 H, s), 2.59 - 2.79 (4 H, m), 3.32 - 3.40 (2 H, m), 4. 97 - 5.05 (1 H, m), 7. 18 - 7.21 (1 H, m), 7.21 - 7.24 (1 H, m), 7.37 (1 H, d, *J*=7.8 Hz), 10.32 (1 H, br s) |
| 3 | - | ESI+; 421.3 |
| | | ¹H NMR (500 MHz, CD₃0D) δ ppm 1. 39 (3 H, t, *J*=7.3 Hz), 1.92 - 2.21 (6 H, m), 2. 21 (3 H, s), 2.91 - 3.09 (3 H, m), 3. 18 - 3. 32 (3 H, m), 3.54 - 3. 66 (4 H, m), 4. 97 - 5.07 (1 H, m), 7.29 - 7. 31 (1 H, m), 7.35 - 7.40 (1 H, m), 7.56 (1 H, d, *J*=7.8 Hz) |
| 4 | - | ESI+; 424.3 |
| | | ¹H NMR (500 MHz, DMSO-*d*₆) δ ppm 1. 19 - 1. 36 (3 H, m), 1.43 - 1. 54 (1 H, m), 1.61 - 1. 74 (2 H, m), 1.86 - 1.94 (1 H, m), 1.96 - 2.03 (4 H, m), 3. 38 - 3.47 (1 H, m), 3.54 - 3.66 (2 H, m), 3.78 (1 H, ddd, *J*=11.0, 5.2, 3. 2 Hz), 3.92 (1 H, ddd, *J*=11.1, 8.0, 3. 3 Hz), 4. 25 - 4. 33 (1 H, m), 4. 63 (1 H, d, *J*=5.4 Hz), 4. 67 (1 H, d, *J*=15.0 Hz), 4.80 (1 H, d, *J*=15.0 Hz), 7.20 - 7.22 (1 H, m), 7.22 - 7.26 (1 H, m), 7.40 (1 H, d, *J*=7.5 Hz), 10. 38 (1 H, br s) |
| 5 | - | ESI+; 379.2 |
| | | ¹H NMR (500 MHz, DMSO-*d*₆) δ ppm 1.95 (3 H, s), 2. 18 - 2. 32 (2 H, m), 2.97 - 3.11 (2 H, m), 3.25 - 3.37 (2 H, m), 3.42 - 3.52 (1 H, m), 3.64 - 3.72 (1 H, m), 4.87 (1 H, dd, *J*=10.4, 8.9 Hz), 7.18 - 7.24 (2 H, m), 7.36 (1 H, d, *J*=7.6 Hz), 7.95 (1 H, s), 10.47 (1 H, br s) |

**[Table 5-2]**

| Ex | Syn | DAT |
|---|---|---|
| 6 | - | ESI+; 385.3 |
| | | ¹H NMR (500 MHz, DMSO-*d*₆) δ ppm 1.41 - 1.53 (1 H, m), 1. 54 - 1. 65 (1 H, m), 1.67 - 1.75 (1 H, m), 1. 76 - 1. 87 (2 H, m), 1. 93 (1 H, t, *J*=10.5 Hz), 1.98 (3 H, s), 2. 19 (3 H, s), 2.72 (1 H, d, *J*=10.4 Hz), 2.95 (1 H, dd, *J*=10.2, 3.7 Hz), 3.42 - 3.51 (2 H, m), 3.71 - 3.79 (5 H, m), 4.56 (1 H, tt, *J*=11.3, 4.0 Hz), 4.63 - 4.73 (2 H, m), 6.46 - 6.51 (2 H, m), 7.06 - 7. 10 (1 H, m), 9. 67 (1 H, s) |
| 7 | - | ESI+; 437.4 |
| | | ¹H NMR (400 MHz, CDCl₃) δ ppm 1.31 - 1.35 (1 H, m) 1.40 - 1. 52 (2 H, m) 1.65 - 1.74 (1 H, m) 1.80 - 1.86 (2 H, m) 1.96 - 2.00 (1 H, m) 2. 17 - 2. 19 (1 H, m) 2.32 (3 H, s) 2.54 (3 H, s) 2.71 - 2.74 (1 H, m) 2.98 - 3.09 (1 H, m) 3.36 - 3.44 (1 H, m) 3.51 - 3.57 (1 H, m) 3.67 - 3.71 (2 H, m) 3.82 - 3.86 (1 H, m) 4. 34 - 4.45 (1 H, m) 7.16 (1 H, d, *J*=8.0 Hz) 7.33 - 7.35 (2 H, m) |
| 8 | 1 | ESI+; 435. 1 |
| 9 | 1 | ESI+; 449.4 |
| 10 | 1 | ESI+; 423.3 |
| 11 | 1 | ESI+; 353.4 |
| 12 | 1 | ESI+; 422. 1 |
| 13 | 1 | ESI+; 407.3 |
| 14 | 1 | ESI+; 421. 3 |
| 15 | 1 | ESI+; 393.3 |
| 16 | 1 | ESI+; 423. 1 |
| 17 | 1 | ESI+; 421. 4 |
| 18 | 1 | ESI+; 408. 1 |
| 19 | 1 | ESI+; 390.3, 392.3 |
| 20 | 1 | ESI+; 354. 2 |
| 21 | 1 | ESI+; 438.2 |
| 22 | 1 | ESI+; 403. 2 |
| 23 | 1 | ESI+; 513. 3 |
| 24 | 1 | ESI+; 440.1 |
| 25 | 1 | ESI+; 439.3 |
| 26 | 1 | ESI+; 473.2 |
| 27 | - | ESI+; 437.4 |

**[Table 5-3]**

| Ex | Syn | DAT |
|---|---|---|
| 28 | - | ESI+; 406.5 |
| | | ¹H NMR (500 MHz, DMSO-*d*₆) δ ppm 1. 15 - 1.37 (3 H, m), 1.39 - 1.57 (1 H, m), 1.61 - 1.80 (4 H, m), 1.92 (3 H, s), 1.93 - 2.02 (2 H, m), 2.53 - 2.74 (2 H, m), 3.25 - 3.41 (2 H, m), 3. 56 - 3.69 (1 H, m), 4.47 (1 H, d, *J*=5.0 Hz), 4.75 (1 H, br s), 6.66 - 6.71 (2 H, m), 7. 15 - 7. 21 (1 H, m), 7.23 (1 H, t, *J*=74. 2 Hz), 10.11 (1 H, br s) |
| 29 | - | ESI+; 408.5 |
| | | ¹H NMR (500 MHz, DMSO-*d*₆) δ ppm 1.63 - 1.71 (1 H, m), 1.72 - 1.83 (2 H, m), 1.90 - 2.01 (4 H, m), 2.57 - 2.67 (1 H, m), 2.67 - 2.75 (1 H, m), 3.07 (1 H, t, *J*=10.3 Hz), 3.27 - 3.43 (3 H, m), 3.70 - 3.80 (1 H, m), 3.84 - 3.93 (2 H, m), 4.86 - 4.98 (2 H, m), 6.67 - 6.71 (2 H, m), 7.16 - 7.20 (1 H, m), 7.23 (1 H, t, *J*=74.2 Hz), 10.10 (1 H, br s) |
| 30 | - | ESI+; 422.3 |
| | | ¹H NMR (500 MHz, DMSO-*d*₆) δ ppm 1.21 - 1.35 (3 H, m), 1.43 - 1.54 (1 H, m), 1.60 - 1.74 (2 H, m), 1.86 - 1.94 (1 H, m), 1.96 -2.03 (4H m), 3.32 - 3.42 (1H, m), 3.50 - 3.64 (2 H, m), 3.77 (1 H, ddd, *J*=11.0, 5.4, 3.1 Hz), 3.90 (1 H, ddd, *J*=11.0, 7.9, 3.1 Hz), 4.21 - 4.29 (1 H, m), 4.63 - 4.69 (2 H, m), 4.77 (1 H, d, *J*=14.8 Hz), 6.68 - 6.73 (2 H, m), 7.19 - 7.23 (1 H, m), 7.25 (1 H, t, *J*=74.1 Hz), 10.09 (1 H, br s) |
| 31 | - | ESI+; 433.2 |
| 32 | - | ESI+; 451.2 |
| 33 | - | ESI+; 436. 2 |
| 34 | - | ESI+; 471. 2 |
| 35 | - | ESI+; 380. 4 |

**[Table 5-4]**

| Ex | Syn | DAT |
|---|---|---|
| 36 | - | ESI+; 423.3 |
| 37 | - | ESI+; 423.2 |
| 38 | - | ESI+; 423.3 |
| | | ¹H NMR (400 MHz, CD₃0D) δ ppm 1.39 - 1.46 (3 H, m) 1.57 - 1.65 (1 H, m) 1.75 - 1.80 (2 H, m) 2.01 - 2.17 (5 H, m) 3.02 - 3.06 (1 H, m) 3.16 - 3.22 (1 H, m) 3.51 - 3.62 (2 H, m) 3.73 - 3.80 (1 H, m) 4.02 - 4. 15 (2 H, m) 4.27 - 4.33 (1 H, m) 7. 16 (1 H, s) 7.24 (1 H, d, *J*=7.8 Hz) 7.38 (1 H, d, *J*=7.8 Hz) |
| 39 | - | ESI+; 507.4 |
| 40 | - | ESI+; 465.3 |
| 41 | - | ESI+; 451.4 |
| 42 | 41 | ESI+; 451. 3 |
| 43 | - | ESI+; 376.3 |
| 44 | - | ESI+; 419. 3 |
| 45 | - | ESI+; 377.2 |
| | | ¹H NMR (500 MHz, CD₃0D) δ ppm 2. 17 (3 H, s), 2.36 - 2.53 (2 H, m), 3.31 - 3.40 (2 H, m), 3.40 - 3.51 (2 H, m), 3.77 - 3.86 (1 H, m), 3.95 - 4.04 (1 H, m), 4.84 - 4.94 (1 H, m), 6.77 - 6.79 (1 H, m), 6.83 (1 H, dd, *J*=8.5, 2.3 Hz), 6.92 (1 H, t, *J*=73.5 Hz), 7. 34 (1 H, d, *J*=8.5 Hz) |

### INDUSTRIAL APPLICABILITY

The compound of formula (I) or a salt thereof has an inhibitory effect on NLRP3 inflammasome activation, and should be able to be used as prophylactic and/or therapeutic agents for inflammatory diseases and/or neurodegenerative diseases.

## Claims

1. A compound of formula (I): 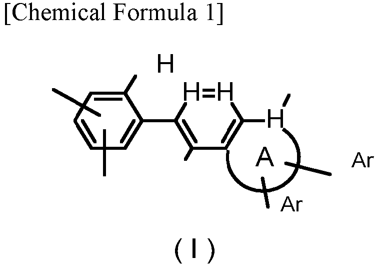
or a salt thereof, wherein
ring A is 5- to 7-membered nitrogen-containing partially unsaturated heterocycle,
R^{1a} and R^{1b} are each independently H, C₁₋₆ alkyl, -O-C₁₋₆ alkyl, halogeno C₁₋₆ alkyl, -O-halogeno C₁₋₆ alkyl, C₃₋₆ cycloalkyl, -O-C₃₋₆ cycloalkyl, or halogen,
R² is H or C₁₋₆ alkyl,
R^{3a} and R^{3b} are each independently H, C₁₋₆ alkyl, -O-C₁₋₆ alkyl, -C₁₋₆ alkylene-optionally substituted aryl, oxo, -C(=O)-C₁₋₆ alkyl, or -S(=O)₂-C₁₋₆ alkyl,
R⁴ is C₁₋₆ alkyl substituted with one to three R⁵, C₃₋₈ cycloalkyl optionally substituted with one to four R⁶, 4- to 7-membered saturated heterocyclyl optionally substituted with one to four R⁷, or heteroaryl optionally substituted with one to four R⁸,
R⁵, R⁶, R⁷, and R⁸ are each independently C₁₋₆ alkyl, C₃₋₈ cycloalkyl, halogeno C₁₋₆ alkyl, 4- to 7-membered saturated heterocyclyl, oxo, cyano, -C(=O)-C₁₋₆ alkyl, -S(=O)₂-C₁₋₆ alkyl, - C(=O)-O-C₁₋₆ alkyl, -C(=O)-NR⁹R¹⁰, -NR⁹R¹⁰, -C₁₋₆ alkylene-OR¹¹, or -OR¹¹,
R⁹ and R¹⁰ are each independently H, C₁₋₆ alkyl, or -C(=O)-C₁₋₆ alkyl, and
R¹¹ is H, C₁₋₆ alkyl, or -C(=O)-C₁₋₆ alkyl.

2. The compound or salt according to claim 1, wherein the formula (I) is formula (Ia), (Ib), (Ic), (Id), (Ie), (If), or (Ig): 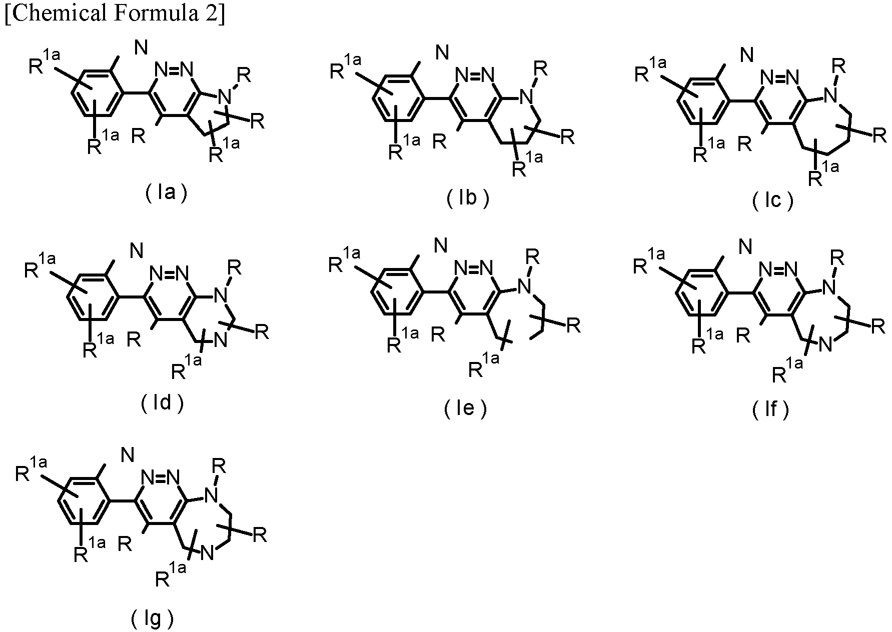 wherein
R^{1a} and R^{1b} are each independently H, C₁₋₆ alkyl, -O-C₁₋₆ alkyl, halogeno C₁₋₆ alkyl, -O-halogeno C₁₋₆ alkyl, or halogen,
R² is H or C₁₋₆ alkyl,
R^{3a} and R^{3b} are each independently H, C₁₋₆ alkyl, -O-C₁₋₆ alkyl, -C₁₋₆ alkylene-optionally substituted aryl, oxo, or -C(=O)-C₁₋₆ alkyl,
R⁴ is C₁₋₆ alkyl substituted with one R⁵, C₃₋₈ cycloalkyl optionally substituted with one to two R⁶, 4- to 7-membered saturated heterocyclyl optionally substituted with one to two R⁷, or unsubstituted heteroaryl,
R⁵ is -NR⁹R¹⁰ or -OR¹¹,
R⁶ is C₁₋₆ alkyl, cyano, -NR⁹R¹⁰, or -OR¹¹,
R⁷ is C₁₋₆ alkyl, C₃₋₈ cycloalkyl, halogeno C₁₋₆ alkyl, 4- to 7-membered saturated heterocyclyl, oxo, -C(=O)-C₁₋₆ alkyl, -S(=O)₂-C₁₋₆ alkyl, -C(=O)-O-C₁₋₆ alkyl, -C(=O)-NR⁹R¹⁰, -C₁₋₆ alkylene-OR¹¹, or -OR¹¹,
R⁹ is H and R¹⁰ is H, C₁₋₆ alkyl, or -C(=O)-C₁₋₆ alkyl, and
R¹¹ is H, C₁₋₆ alkyl, or -C(=O)-C₁₋₆ alkyl.

3. The compound or salt thereof according to claim 2, wherein the formula (I) is formula (Ia), (Ib), (Ie), or (If),
R^{1a} is -O-C₁₋₆ alkyl, halogeno C₁₋₆ alkyl, or -O-halogeno C₁₋₆ alkyl and R^{1b} is H,
R² is C₁₋₆ alkyl,
R^{3a} is H and R^{3b} is H or C₁₋₆ alkyl,
R⁴ is C₃₋₈ cycloalkyl substituted with one R⁶ or 4- to 7-membered saturated heterocyclyl substituted with one R⁷,
R⁶ is -OR¹¹,
R⁷ is C₁₋₆ alkyl, oxo, or -OR¹¹, and
R¹¹ is H.

4. The compound or salt thereof according to claim 1, wherein the compound is selected from the group consisting of:
2-{8-[(1R,2R)-2-hydroxycyclohexyl]-4-methyl-5,6,7,8-tetrahydropyrido[2,3-c]pyridazin-3-yl}-5-(trifluoromethyl)phenol,
2-{4-methyl-8-[(3R)-1-methylpiperidin-3-yl]-5,6,7,8-tetrahydropyrido[2,3-c]pyridazin-3-yl}-5-(trifluoromethyl)phenol;
2-{8-[(3R)-1-ethylpiperidin-3-yl]-4-methyl-5,6,7,8-tetrahydropyrido[2,3-c]pyridazin-3-yl}-5-(trifluoromethyl)phenol;
5-(difluoromethoxy)-2-{8-[(1R,2R)-2-hydroxycyclohexyl]-4-methyl-5,6,7,8-tetrahydropyrido[2,3-c]pyridazin-3-yl}phenol;
(3S,4R)-4-{3-[4-(difluoromethoxy)-2-hydroxyphenyl]-4-methyl-6,7-dihydropyrido[2,3-c]pyridazin-8(5H)-yl}oxan-3-ol;
2-{9-[(1R,2R)-2-hydroxycyclohexyl]-4-methyl-5,7,8,9-tetrahydropyridazino[3,4-e][1,4]oxaazepin-3-yl}-5-(trifluoromethyl)phenol;
5-(difluoromethoxy)-2-{9-[(1R,2R)-2-hydroxycyclohexyl]-4-methyl-5,7,8,9-tetrahydropyridazino[3,4-e][1,4]oxaazepin-3-yl}phenol;
(3 S)-3-{3-2-hydroxy-4-(trifluoromethyl)phenyl]-4-methyl-5,6-dihydro-7H-pyrrolo[2,3 - c]pyridazin-7-yl}pyrrolidin-2-one;
5-methoxy-2-{4-methyl-9-[(3R)-1-methylpiperidin-3-yl]-5,7,8,9-tetrahydropyridazino[3,4-e][1,4]oxaazepin-3-yl}phenol;
2-{9-[(1R,2R)-2-hydroxycyclohexyl]-4,6-dimethyl-6,7,8,9-tetrahydro-5H-pyridazino[3,4-e][1,4]diazepin-3-yl}-5-(trifluoromethyl)phenol;
2-{9-[(1R,2R)-2-hydroxycyclohexyl]-4-methyl-6,7,8,9-tetrahydro-5H-pyridazino[3,4-e][1,4]diazepin-3-yl }-5-(trifluoromethyl)phenol; and
(3S)-3-{3-[4-(difluoromethoxy)-2-hydroxyphenyl]-4-methyl-5,6-dihydro-7H-pyrrolo[2,3-c]pyridazin-7-yl}pyrrolidin-2-one.

5. A pharmaceutical composition comprising the compound or salt thereof according to claim 1 and one or more pharmaceutically acceptable excipients.

6. The pharmaceutical composition according to claim 5, which is an inhibitor of NLRP3 inflammasome activation.

7. The pharmaceutical composition according to claim 5, which is a pharmaceutical composition for prevention and/or treatment of an inflammatory and/or neurodegenerative disease.

8. Use of the compound or salt thereof according to claim 1 for the manufacture of a pharmaceutical composition for prevention and/or treatment of an inflammatory and/or neurodegenerative disease.

9. Use of the compound or salt thereof according to claim 1 for prevention and/or treatment of an inflammatory and/or neurodegenerative disease.

10. The compound or salt thereof according to claim 1 for use in prevention and/or treatment of an inflammatory and/or neurodegenerative disease.

11. A method of preventing and/or treating an inflammatory and/or neurodegenerative disease, comprising administering to a subject an effective amount of the compound or salt thereof according to claim 1.
